# EUROPEAN PATENT APPLICATION

(11) **EP 4 571 743 A1**
(43) Date of publication of application: **18.06.2025**
(21) Application number: 24219175.7
(22) Date of filing: 11.12.2024
(51) Int. Cl.: G10L 25/66, G09B 19/04, A61B 5/00

(54) **AUTOMATED GENERATION OF TARGETED FEEDBACK USING SPEECH CHARACTERISTICS EXTRACTED FROM AUDIO SAMPLES TO ADDRESS SPEECH DEFECTS**

(30) Priority: 12.12.2023 US 202363609270 P; 03.12.2024 US 202418967074
(71) Applicant: Click Therapeutics, Inc., New York, NY 10013 (US)
(72) Inventor: MORSE, William, New York, 10013 (US); MURSKY-FULLER, Jesse, New York, 10013 (US)
(74) Representative: Marks & Clerk LLP

(57) **Abstract**

Provided herein are systems and methods for providing instructions for speech based on speech classifications of verbal communications from users. A computing system can generate speech characteristics for a first verbal communication using a first audio sample from a user. The computing system can determine, from a plurality of speech classifications, a first speech classification for the first verbal communication based on the speech characteristics. The computing system can select, from a plurality of actions, an action comprising modifying one or more of the speech characteristics to define an utterance for the user based on the first speech classification. The computing system can provide an instruction presenting a message to prompt the user to perform the utterance defined by the action selected from the plurality of actions. The efficacy of the medication that the user is taking to address the condition may be increased.

## Description

### CROSS REFERENCES TO RELATED APPLICATIONS

The present application claims the benefit of and priority to U.S. Non-Provisional Patent Application No. 18/967,074, titled "Automated Generation of Targeted Feedback Using Speech Characteristics Extracted from Audio Samples to Address Speech Defects," filed December 3, 2024, which claims the benefit of and priority to U.S. Provisional Patent Application No. 63/609,270, titled "Automated Generation of Targeted Feedback Using Speech Characteristics Extracted from Audio Samples to Address Speech Defects," filed December 12, 2023, which is incorporated herein by reference in its entirety.

### BACKGROUND

Speech defects may include impairments or deviations in the production of speech sounds that may impact an individual's ability to communicate effectively. These defects can manifest in various ways and may affect the clarity, fluency, or overall intelligibility of speech. There are a number of different classifications of speech defects. Articulation disorders may result in distorted, substituted, or omitted sounds, making speech less clear. Fluency disorders involve disruptions in the natural flow of speech, leading to stuttering, mumbling, repetitions, prolongations, or blocks in the production of sounds, among others. Vocal disorders may affect the quality, pitch, or volume of the voice. These speech defects can have a wide array of causes, including various types of neurological disorders such as alogia and blunted speech affect.

The daily lives of individuals with speech defect may be negatively impacted by their condition. For example, speech defects may lead to unclear or distorted speech, making it difficult for others to understand the individual, leading to frequent misunderstanding and inability to convey information effectively to others. Furthermore, individuals with speech defects may avoid social situations or withdraw from conversations due to fear of being misunderstood or judged. In addition, persistent speech difficulties may lead to emotional stress and anxiety. Fear of judgment, ridicule, or rejection may contribute to heightened emotional distress, further exacerbating communication challenges.

Speech intervention techniques (e.g., under the guidance of a speech-language pathologists) may be used to treat speech defects. Treating speech defects in patients can prove difficult due to the diversity of the speech defects. Each speech defect has its own unique set of characteristics, and each patient has a unique cadence to their voice which makes treatment difficult. For example, the treatment for an individual with monotonous speech may be different from the treatment for an individual with stuttering. Moreover, without an individually tailored solution, treatments for individuals may be prolonged due to the differences between each patient regarding their speech. Therefore, therapies may prove ineffective to treat individuals with speech defects without a therapy personalized to the specific speech of the individual.

Furthermore, subjectivity in assessing and treating speech and language difficulties may also pose challenges in providing effective therapy to individuals with speech defects and impediments. For instance, the assessment of speech and language disorders often involves subjective judgment by speech-language pathologists, even with the aid of computer-assisted therapy techniques. Different pathologists may interpret speech samples or performance in therapy sessions differently, leading to variations in diagnoses and treatment plans. Not to mention, it may be difficult for an individual to gain access to pathologists to receive the therapy.

### SUMMARY

To address these and other technical challenges, the digital therapeutic application described herein can be provided to a user to address speech defects and impediments, by allowing the user to self-record audio samples of verbal communication and then quickly receiving from the digital therapeutic application a direct, targeted, real-time feedback with corrective actions to address their specific speech defects and impediments. In the context of a digital therapeutics application, the output containing a targeted treatment and instructions on modifying the user's speech can result in the real-time delivery of user-specific interventions improving the user's adherence to the treatment over time. Moreover, as the digital therapeutics application can provide a more targeted individualized response aimed to address the user's particular speech, then the user's time, effort, and computing resources (e.g., processing and memory) that would have otherwise been consumed in less effective speech interventions can be saved. Further, the digital therapeutic application may allow the user to self-practice repeatedly anywhere by themselves and also receive an objective measure without the influence of the subjective, inconsistent opinion of different pathologists.

The application can prompt the user to record a verbal communication through a microphone of the user device. For instance, the user may be directed to utter a set of words or a phrase displayed on a graphical user interface of the application on the device. Upon obtaining an audio sample of the verbal communication, the application (or a service interfacing with the application) can process the audio sample and determine a set of speech characteristics of the verbal communication of the user. The speech characteristics can include, for example, respiration, phonation, articulation, resonance, prosody, pitch, jitter, shimmer, and rhythm, among others. For each speech characteristic, the application can calculate a score indicating an objective degree of severity of the corresponding speech characteristic relative to understandable speech. The application can also use a video recording obtained concurrently with the audio sample to determine non-verbal characteristics of the user accompanying the verbal communication. The non-verbal characteristics can include, for example, gesture, facial expression, or eye contact, among others.

Using the set of speech characteristics, the digital therapeutic application can classify the verbal communication of the user. The classification can identify whether the verbal communication is understandable or is not understandable and can identify the speech as having mumbling, lisp, or dysarthria, among others. The classification may be based on any number of functions of the set of speech characteristics identified from the audio sample, and may be augmented by the non-verbal characteristics determined from the video recording. For example, the application can apply the speech characteristics to a machine learning model to determine a classification for the verbal speech. The model may have been trained using a training dataset in accordance with supervised learning techniques. The training dataset can include a set of examples, with each example containing a sample audio recording of a verbal communication by another individual and an annotation indicating the classification of the verbal communication. Other functions can be used to classify the user's speech.

Based on the classification of the verbal communication, the digital therapeutic application can select an action for the user with respect to the user's utterance of the words in the verbal communication. When the classification indicates that the verbal communication is understandable, the application can identify that the user is to maintain how the user uttered the words. In contrast, when the classification indicates that the verbal communication is not understandable, the application can select a corrective action to modify the user's utterance. The corrective action can be related to one or more of the speech characteristics and the degrees of severity with respect to the speech characteristics. For instance, if the verbal communication by the user is classified as mumbling, the application can select an action to increase articulation and include pacing when uttering to address the mumbling. In addition, the application can identify a causal factor (or diagnosis) of the classification based on the speech characteristics. For example, when the user's verbal communication is classified as not understandable, the application can identify the speech characteristics with the highest degrees of severity as part of the causal factor resulting in the classification.

The digital therapeutic application can generate an instruction with a message to indicate to the user to perform the utterance for the classification. The instruction can also identify the scores for each speech characteristic as well as the classification itself. This way, the user can see visually the extent of each speech characteristic in relation to the classification of the user's speech. In addition, the application can generate a modified version of the audio sample from the user, with the corrective action applied. The modified version can include audio of the verbal communication as how the utterances of words should sound, such that others can understand the words therein. The application can use text-to-speech (TTS) techniques to manipulate the audio sample from the user to output the modified version. The application can provide the instruction to the user by displaying the message via the graphical user interface on the user's device. The application can also present a playback of the modified version of the audio sample. In this manner, the user may be able to hear their own voice with the corrective actions identified for the user's particular speech classifications, and subsequently adjust user's utterances of words.

The digital therapeutic application can repeat this process by receiving additional audio samples of the user's verbal communication and providing instructions to modify utterance of the user's speech. The application can determine a progression metric for the user over multiple audio samples across time. The application can also display the progression metric to the user to provide an indication of whether the user's speech has improved over time. By repetitively taking audio samples and providing instructions regarding speech, the user can be informed about the corrective steps to take in an effort to improve the user's speech. In addition, the user may listen to the modified version of the user's own speech to help realize how the user should sound in order to be understood by others. Moreover, the digital therapeutic application can provide feedback beyond verbal aspects of speech, also providing feedback on the context of the speech (e.g., providing different feedback depending on whether the user is speaking to a friend versus speaking to a manager) and emotion (e.g., identifying flat affect and providing feedback on how to express more emotion), among others.

In this manner, the digital therapeutic application may allow the user to directly record audio samples of verbal communication to quickly receive direct feedback regarding corrective actions to address their speech defects. This functionality can improve the quality of human-computer interaction (HCI) between the user and their device, by providing additional utility for the device. In the context of a digital therapeutics application, the instructions regarding the user's speech can result in the real-time delivery of user-specific interventions to improve the user's adherence to the treatment. Moreover, as the digital therapeutics application can provide a more targeted response aimed to address the user's particular speech, computing resources (e.g., processing and memory) that would have otherwise been consumed in less effective computer-assisted speech interventions can be saved.

Aspects of the present disclosure are directed to systems and methods for providing instructions for speech based on speech classifications of verbal communications from users. One or more processors coupled with memory can identify a first audio sample of a first verbal communication from a user. The one or more processors can generate a first plurality of speech characteristics for the first verbal communication using the first audio sample. The one or more processors can determine, from a plurality of speech classifications, a first speech classification for the first verbal communication based on the first plurality of speech characteristics. The one or more processors can select, from a plurality of actions, an action comprising modifying one or more of the speech characteristics to define an utterance for the user based on the first speech classification. The one or more processors can provide an instruction presenting a message to prompt the user to perform the utterance defined by the action selected from the plurality of actions.

In some embodiments, the one or more processors can determine that the verbal communication is not able to be understood. In some embodiments, the one or more processors can select the action for the user to modify at least one of the first plurality of speech characteristics in the utterance. In some embodiments, the one or more processors can determine that the verbal communication is able to be understood. In some embodiments, the one or more processors can select the action for the user to maintain one or more of the first plurality of speech characteristics in the utterance.

In some embodiments, the one or more processors can generate a score indicating a degree of severity of at least one of the first plurality of the speech characteristics. In some embodiments, the one or more processors can provide the instruction including the message to identify the score for presentation to the user. In some embodiments, the first plurality of speech characteristics can include a corresponding plurality of scores. Each of the plurality of scores can be defined along a scale for a respective speech characteristic.

In some embodiments, the one or more processors can determine the first speech classification based on at least one of (i) an average of the plurality of scores, (ii) a weighted combination of the plurality of scores, (iii) a comparison with a dataset comprised of a second plurality of scores, (iv) a neural network model, or (v) a generative transformer model. In some embodiments, the one or more processors can apply a machine learning (ML) model to the first plurality of speech characteristics. The ML model can be established using a training dataset comprising a plurality of examples, each of the plurality of examples identifying (i) a respective second audio sample of a second verbal communication and (ii) a respective second classification from the plurality of speech classifications.

In some embodiments, the one or more processors can provide the instruction including the message to identify at least one of (i) one or more of the first plurality of speech characteristics and (ii) the action to modify the utterance. In some embodiments, the one or more processors can identify, from a plurality of factors, a factor as causing the first speech classification based on at least one of the first plurality of speech characteristics. In some embodiments, the one or more processors can provide the instruction including the message to identify the factor as the cause of the first speech characteristic.

In some embodiments, the one or more processors can generate, for playback to the user, a second audio sample by modifying the first audio sample in accordance with the action. In some embodiments, the one or more processors can apply a speech synthesis model to the first audio sample and the action to generate the second audio sample. In some embodiments, the one or more processors can determine a second speech classification for a second verbal communication of the user based on a second plurality of speech characteristics. The second plurality of speech characteristics can be generated from a second audio sample identified at a time subsequent to provision of the instruction. In some embodiments, the one or more processors can determine a progress metric based on a comparison between the first speech classification from prior to the instruction and the second speech classification subsequent to the provision of the instruction.

In some embodiments, the first plurality of speech characteristics can include at least one of (i) respiration, (ii) phonation, (iii) articulation, (iv) resonance, (v) prosody, (vii) pitch, (viii) jitter, (ix) shimmer, or (x) rhythm. In some embodiments, the plurality of speech classifications can include at least one of (i) mumbling, (ii) lisp, (iii) dysarthria, (vi) stuttering, or (v) understandable. In some embodiments, the user may be affected by at least one of a speech impairment or a language impairment, and may be undergoing speech therapy at least partially concurrently with the provision of the instruction. In some embodiments, the user may be affected by a disorder associated with a speech impairment and may be on a medication for the disorder at least partially concurrently with the provision of the instruction.

In some embodiments, the one or more processors can identify a first video sample of a first non-verbal communication from the user, at least in partial concurrence with the first verbal communication. In some embodiments, the one or more processors can determine a first plurality of non-verbal characteristics of the first non-verbal communication using the first video sample. The first plurality of non-verbal characteristics can include at least one of a gesture or an eye contact by the user. In some embodiments, the one or more processors can determine the first speech classification based on the first plurality of non-verbal characteristics.

Other aspects are directed to systems and method for providing instructions for speech based on characteristics of verbal communications from users. One or more processors coupled with memory can identify a first audio sample of a first verbal communication from a user. The one or more processors can generate a first plurality of speech characteristics for the first verbal communication using the first audio sample. The one or more processors can select, from a plurality of actions, an action to modify one or more of the first plurality of speech characteristics to define an utterance for the user. The one or more processors can provide an instruction presenting a message to prompt the user to perform the utterance defined by the action selected from the plurality of actions.

In some embodiments, the one or more processors can determine the verbal communication is not able to be understood. In some embodiments, the one or more processors can select the action for the user to modify at least one of the first plurality of speech characteristics in the utterance. In some embodiments, the one or more processors can determine the verbal communication is able to be understood. In some embodiments, the one or more processors can select the action for the user to maintain one or more of the first plurality of speech characteristics in the utterance.

In some embodiments, the one or more processors can generate a score indicating a degree of severity of at least one of the first plurality of the speech characteristics. In some embodiments, the one or more processors can provide the instruction including the message to identify the score for presentation to the user. In some embodiments, the first plurality of speech characteristics can include a corresponding plurality of scores. Each of the plurality of scores can be defined along a scale for a respective speech characteristic. In some embodiments, the first plurality of speech characteristics can include corresponding plurality of scores. Each of the plurality of scores can be defined along a scale for a respective speech characteristic.

In some embodiments, the one or more processors can determine the first speech classification based on at least one of (i) an average of the plurality of scores, (ii) a weighted combination of the plurality of scores, (iii) a comparison with a dataset comprised of a second plurality of scores, (iv) a neural network model, or (v) a generative transformer model. In some embodiments, the one or more processors can apply a machine learning (ML) model to the first plurality of speech characteristics. The ML model can be established using a training dataset comprising a plurality of examples. Each of the plurality of examples can identify (i) a respective second audio sample of a second verbal communication and (ii) a respective second classification from the plurality of speech classifications.

In some embodiments, the one or more processors can provide the instruction including the message to identify at least one of (i) one or more of the first plurality of speech characteristics and (ii) the action to modify the utterance. In some embodiments, the one or more processors can identify, from a plurality of factors, a factor based on at least one of the first plurality of speech characteristics and provide the message to identify the factor. In some embodiments, the one or more processors can generate, for playback to the user, a second audio sample by modifying the first audio sample in accordance with the action. In some embodiments, the one or more processors can apply a speech synthesis model to the first audio sample and the action to generate the second audio sample.

In some embodiments, the one or more processors can determine a second speech classification for a second verbal communication of the user based on a second plurality of speech characteristics. The second plurality of speech characteristics can be generated from a second audio sample identified at a time subsequent to provision of the instruction. In some embodiments, the one or more processors can determine a progress metric based on a comparison between the first speech classification from prior to the instruction and the second speech classification subsequent to the provision of the instruction.

In some embodiments, the first plurality of speech characteristics can include at least one of (i) respiration, (ii) phonation, (iii) articulation, (iv) resonance, (v) prosody, (vii) pitch, (viii) jitter, (ix) shimmer, (x) rhythm, (xi) pacing, or (xii) pausing. In some embodiments, the user may be affected by at least one of a speech impairment or a language impairment, and may be undergoing speech therapy at least partially concurrently with the provision of the instruction. In some embodiments, the user may be affected by a disorder associated with a speech impairment and may be on a medication for the disorder at least partially concurrently with the provision of the instruction.

In some embodiments, the one or more processors can identify a first video sample of a first non-verbal communication from the user, at least in partial concurrence with the first verbal communication. In some embodiments, the one or more processors can determine a first plurality of non-verbal characteristics of the first non-verbal communication using the first video sample. The first plurality of non-verbal characteristics can include at least one of a gesture, a facial expression, or an eye contact by the user. In some embodiments, the one or more processors can determine the action based on the first plurality of non-verbal characteristics.

Other aspects of the present disclosure are directed to systems and methods of ameliorating defect of speech expressiveness in a user in need thereof. One or more processors coupled with memory can obtain a first metric associated with the user prior to completion of at least one of a plurality of sessions. The one or more processors can repeat provision of the plurality of sessions to the user. Each session of the plurality of sessions can include identifying a first audio sample of a first verbal communication from a user; generating a first plurality of speech characteristics for the first verbal communication using the first audio sample; determining, from a plurality of actions, an action to modify one or more of the first plurality of speech characteristics to define an utterance for the user; and providing an instruction presenting a message to prompt the user to perform the utterance defined by the action selected from the plurality of actions. The one or more processors can obtain a second metric associated with the user subsequent to the completion of at least one of the plurality of sessions. Amelioration in the defect of speech expressiveness may occur in the user, when the second metric is (i) decreased from the first metric by a first predetermined margin or (ii) increased from the first metric by a second predetermined margin.

In some embodiments, the user may be diagnosed with a condition comprising at least one of a speech pathology, autism spectrum disorder (ASD), multiple sclerosis, an affective disorder, a neurodegenerative disease, Alzheimer's disease, dementia, Parkinson's disease, or schizophrenia. In some embodiments, the user may be receiving a treatment, at least in partial concurrence with the at least one of the plurality of sessions. The treatment can include at least one of a psychosocial intervention or a medication to address the condition.

In some embodiments, the defect of the speech expressiveness may be caused by the condition. In some embodiments, the user may be an adult aged at least 18 years or older . In some embodiments, the plurality of sessions may be provided over a period of time ranging between 3 days to 6 months. In some embodiments, the first verbal communication of the first audio sample can include an utterance of one or more words by the user. In some embodiments, the first plurality of speech characteristics can include at least one of (i) respiration, (ii) phonation, (iii) articulation, (iv) resonance, (v) prosody, (vii) pitch, (viii) jitter, (ix) shimmer, (x) rhythm, (xi) pacing, or (xii) pausing.

In some embodiments, at least one of the plurality of session can include determining, from a plurality of speech classifications, a first speech classification for the first verbal communication based on the first plurality of speech characteristics and selecting, from a plurality of actions, an action comprising modifying one or more of the speech characteristics to define an utterance for the user based on the first speech classification. The plurality of speech classifications can include at least one of (i) mumbling, (ii) lisp, (iii) dysarthria, (vi) stuttering, or (v) understandable.

In some embodiments, the amelioration in the defect in the speech expressiveness in the user with a speech pathology may occur, when the second metric is decreased from the first metric by the first predetermined margin or when the second metric is increased from the first metric by the second predetermined margin, and wherein the first metric and the second metric are at least one of: Goldman-Fristoe Test of Articulation (GFTA-3) values, Arizona Articulation Proficiency Scale (Arizona-3) values, speech intelligibility index (SII) values, Percentage of Intelligible Words (PIW) values, Percent Intelligible Utterances (PIU) values, Percentage of Intelligible Syllables (PIS) values, Percentage of Consonants Correct (PCC) values, Percentage of Vowels Correct (PVC) values, Percentage of Vowels and Diphthongs Correct (PVC-R) values, Stuttering Severity Instrument (SSI-4) values, Overall Assessment of the Speaker's Experience of Stuttering (OASES) values, maximum phonation time (MPT) values, GRBAS scale values, vocal range profile (VRP) values, voice handicap index (VHI) values, Voice Related Quality of Life (V-RQOL) values, Consensus Auditory-Perceptual Evaluation of Voice (CAPE-V) values, Diadochokinetic Rate (DDK) values, prosody voice screening profile (PVSP) values, Bzoch Hypernasality Scale values, Resonance Severity Index values, nasalance score values, Western Aphasia Battery (WAB) values, Boston Diagnostic Aphasia Examination (BDAE) values, Communicative Effectiveness Index (CETI) values, Apraxia Battery for Adults (ABA-2) values, DDK rate, Percentage of Consonants Correct-Revised (PCC-R) values, Frenchay Dysarthria Assessment (FDA-2) values, or Dysarthria Impact Profile (DIP) values.

In some embodiments, the amelioration in the expressiveness of speech in the user with ASD may occur, when the second metric is decreased from the first metric by the first predetermined margin or when the second metric is increased from the first metric by the second predetermined margin, and wherein the first metric and the second metric are at least one of Autism Diagnostic Observation Schedule (ADOS) values, Test of Pragmatic Language (TOPL-2) values, CETI values, Social Responsiveness Scale (SRS-2) values, Comprehensive Assessment of Spoken Language (CASL-2) values, or Functional Communication Profile (FCP-R) values. In some embodiments, the amelioration in the expressiveness of speech in the user with multiple sclerosis may occur, when the second metric is decreased from the first metric by the first predetermined margin or when the second metric is changed from the first metric by the second predetermined margin, and wherein the first metric and the second metric are at least one of WAB values, BDAE values, CETI values, ABA-2 values, DDK rate values, PCC-R values, FDA-2 values, or DIP values.

In some embodiments, the amelioration in the expressiveness of speech in the user with affective disorder occurs, when the second metric is decreased from the first metric by the first predetermined margin or when the second metric is changed from the first metric by the second predetermined margin, and wherein the first metric and the second metric are at least one of Hamilton Rating Scale for Depression (HAM-D) values. In some embodiments, the amelioration in the expressiveness of speech in the user with schizophrenia may occur, when the second metric is decreased from the first metric by the first predetermined margin or when the second metric is changed from the first metric by the second predetermined margin, and wherein the first metric and the second metric are at least one of Motivation and Pleasure Scale-Self Report (MAP-SR) values, Social Effort and Conscientiousness Scale (SEACS) Social Effort values, SEACS Social Conscientiousness values. In some embodiments, the first metric may be determined based on a corresponding speech classification of a plurality of speech classifications in a first session of the plurality of sessions. The second metric may be determined based on the respective first respective speech classification in a second session of the plurality of sessions.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for providing instructions for speech based on speech classifications of verbal communications from users, in accordance with an illustrative embodiment;
FIG. 2 depicts a block diagram of a process of parsing audio samples in the system for providing instructions, in accordance with an illustrative embodiment;
FIG. 3 depicts a block diagram of a process of applying classification of speech in the system for providing instructions, in accordance with an illustrative embodiment;
FIG. 4 depicts a block diagram of a process of providing feedback in the system for providing instructions, in accordance with an illustrative embodiment;
FIG. 5 depicts a block diagram of a process of tracking progress of users in the system for providing instructions, in accordance with an illustrative embodiment;
FIG 6A-6F depict screenshots of user interfaces in the system for providing instructions for speech based on speech classifications of verbal communications from users in accordance with illustrative embodiments;
FIG. 7 depict a flow diagram of a method of providing instructions for speech based on speech classifications of verbal communications from users, in accordance with an illustrative embodiment;
FIG. 8 depicts a flow diagram of a method of ameliorating defect of speech expressiveness in a user in need thereof, in accordance with an illustrative embodiment;
FIG. 9 is a block diagram of a study design for testing an application for ameliorating defects of speech expressiveness in users in accordance with an illustrative embodiment; and
FIG. 10 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:
Section A describes systems and methods for providing instructions for speech based on speech classifications of verbal communications from users;
Section B describes methods of ameliorating defect of speech expressiveness in a user in need thereof; and
Section C describes a network and computing environment which may be useful for practicing embodiments described herein.

### A. Systems and Methods for Providing Instructions for Speech Based on Speech Classifications of Verbal Communications from Users

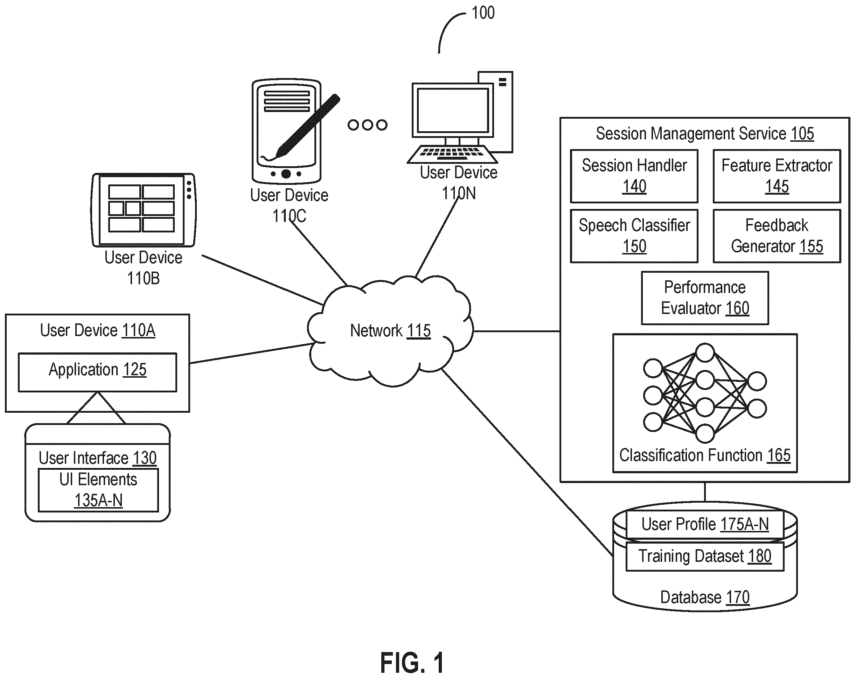

Referring now to FIG. 1, depicted is a block diagram of a system 100 for providing instructions for speech based on speech classifications of verbal communications from users. In an overview, the system 100 may include at least one session management service 105 and a set of user devices 110A-N (hereinafter generally referred to as user devices 110), communicatively coupled with one another via at least one network 115. At least one of the user devices 110 (e.g., the first user device 110A as depicted) may include at least one application 125. The application 125 may include or provide at least one user interface 130 with one or more user interface (UI) elements 135A-N (hereinafter generally referred to as UI elements 135). The session management service 105 may include at least one session handler 140, at least one speech classifier 150, at least one feature extractor 145, at least one feedback generator 155, at least one performance evaluator 160, and at least one classification function 165, among others. The session management service 105 may include or have access to at least one database 170. The database 170 may store, maintain, or otherwise include one or more user profiles 175A-N (hereinafter generally referred to as user profiles 175) and a training dataset 180. The functionalities of the application 125 on the user device 110 may be performed in part on the session management service 105, and vice-versa. Each of the components of the system 100 can be implemented using the computing system a described in Section C.

In further detail, the session management service 105 (sometimes herein generally referred to as a messaging service) may be any computing device comprising one or more processors coupled with memory and software and capable of performing the various processes and tasks described herein. The session management service 105 may be in communication with the one or more user devices 110 and the database 170 via the network 115. The session management service 105 may be situated, located, or otherwise associated with at least one computer system. The computer system may correspond to a data center, a branch office, or a site at which one or more computers corresponding to the session management service 105 are situated.

Within the session management service 105, the session handler 140 can manage a session and can receive an audio sample of a verbal communication from a user. The feature extractor 145 can generate a set of speech characteristics from the audio sample. The speech classifier 150 can determine a classification of the verbal communication of the user using the speech characteristics. The feedback generator 155 can generate a modified version of an audio sample to provide to the user. The performance evaluator 160 can track a progress of the user through multiple sessions over time.

The classification function 165 can be used to determine a classification of the speech from the user based on the set of speech characteristics. In some embodiments, the classification function 165 may include a combination (e.g., an average) of scores corresponding to the set of speech characteristics. In some embodiments, the classification function 165 may include a weighted combination of scores corresponding to the set of speech characteristics. In some embodiments, the classification function 165 can be a mapping between a combination of scores of speech characteristics and speech classifications.

In some embodiments, the classification function 165 can include a set of weights arranged across a set of layers in accordance with a machine learning (ML) model. The architecture for the machine learning model can include, for example, a deep learning neural network (e.g., convolutional neural network architecture), a regression model (e.g., linear or logistic regression model), a random forest, a support vector machine (SVM), a clustering algorithm (e.g., k-nearest neighbors), or a Naive Bayesian model, among others. In general, the classification function 165 may have at least one input and output. The input and output may be related via a set of weights. The input may be an audio sample, a set of speech classifications, or a set of acoustic features, among others. The output may be at least one of a classification or an action, among others. The machine learning model of the classification function 165 can be trained using the training dataset 180. The training dataset 180 may include a set of examples. Each example can include an input (e.g., an audio sample, a set of speech classifications, or a set of acoustic features) and an expected output (e.g., the classification or the action).

In some embodiments, the classification function 165 can include the set of weights arranged across a set of layers in accordance with a transformer architecture. The transformer architecture may receive inputs in the form of a set of strings (e.g., from a text input) to output content in one or more modalities (e.g., in the form of text strings, audio content, images, video, or multimedia content). The generative transformer model may be a machine learning model in accordance with a transformer model (e.g., generative pre-trained model or bidirectional encoder representations from transformers). Under the architecture, the classification function 165 can include at least one tokenization layer (sometimes referred to herein as a tokenizer), at least one input embedding layer, at least one position encoder, at least one encoder stack, at least one decoder stack, and at least one output layer, among others, interconnected with one another (e.g., via forward, backward, or skip connections). The generative transformer model can be a large language model (LLM), a text-to-image model, a text-to-audio model, or a text-to-video model, among others. The generative transformer model can be trained using the training dataset 180. The training dataset 180 may include a set of examples (e.g., in the form of a corpus). Each example can include an input (e.g., an audio sample, a set of speech classifications, or a set of acoustic features) and an expected output (e.g., the classification or the action).

The user device 110 (sometimes herein referred to as an end user computing device) may be any computing device comprising one or more processors coupled with memory and software and capable of performing the various processes and tasks described herein. The user device 110 may be in communication with the session management service 105 and the database 170 via the network 115. The user device 110 may be a smartphone, other mobile phone, tablet computer, wearable computing device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The application 125 executing on the user device 110 may be a digital therapeutics application. The application 125 may provide a session (sometimes referred to herein as a therapy session) to address at least one speech defect of the user. The speech defect of the user may include, for example, articulation disorders (e.g., substitutions or omissions), fluency disorders (e.g., mumbling or stutter), voice disorders (e.g., hoarseness, nasality, pitch disorders, or volume disorder), resonance disorders (e.g., hypernasality or hyponasality), apraxia of speech, aphasia, or dysarthria, among others. The user of the application 125 may be diagnosed with or at risk of a condition. The condition may include any number of disorders that cause the speech defect in the user. The condition may include, for example, a speech impediment or pathology, a neurological disorder (e.g., schizophrenia with positive or negative symptoms, mild cognitive impairment, autism spectrum disorder (ASD), a neurodegenerative disease, Alzheimer's disease, dementia, Parkinson's disease, or multiple sclerosis), an affective disorder (e.g., major depressive disorder, anxiety disorder, bipolar disorder or a post-traumatic stress disorder (PTSD)), or drug abuse, among others.

The user of the application may be affected by at least one of a speech impairment (e.g., articulation disorder, fluency disorder, voice disorder, resonance disorder, apraxia of speech, aphasia, or dysarthria), or a language impairment (e.g., specific language impairment (SLI), expressive language disorder, receptive language disorder, and language processing disorder). The user may be undergoing speech therapy (e.g., articulation therapy, phonological awareness activity, voice therapy, and fluency shaping therapy) at least partially concurrently with the use of the application 125. The user may be at least partially concurrently taking medication to address the disorder associated with the speech defect or speech impairment, at least partially concurrently with the use of the application 125. The medication may be at least orally administered, intravenously administered, or topically applied. For example, for schizophrenia, the user may include a typical antipsychotic (e.g., haloperidol, chlorpromazine, fluphenazine, perphenazine, loxitane, thioridazine, or trifluoperazine) or an atypical antipsychotic (e.g., aripiprazole, risperidone, clozapine, quetiapine, olanzapine, ziprasidone, lurasidone, paliperidone, or iclepertin), among others. For affective disorders (e.g., PTSD or depression), the user may be on a serotonin reuptake inhibitor (SRI) or a mood-stabilizing drug (e.g., lithium, valproic acid, divalproex sodium, carbamazepine, or lamotrigine), among others. The application 125 may increase the efficacy of the medication that the user is taking to address the condition. The interventions for the disorder can include a psychosocial intervention, such as psychoeducation, group therapy, cognitive-behavioral therapy (CBT), early intervention for first-episode psychosis (FEP), cognitive rehabilitation, or educational plans, among others.

The application 125 can include, present, or otherwise provide a user interface 130 including the one or more user interface elements 135A-N (hereinafter generally referred to as UI elements 135) to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide a session (sometimes referred to herein as a therapy session) via the user interface 130 to address the speech defect.

The database 170 may store and maintain various resources and data associated with the session management service 105 and the application 125. The database 170 may include a database management system (DBMS) to arrange and organize the data maintained thereon, as the user profiles 175, among others. The database 170 may be in communication with the session management service 105 and the one or more user devices 110 via the network 115. While running various operations, the session management service 105 and the application 125 may access the database 170 to retrieve identified data therefrom. The session management service 105 and the application 125 may also write data onto the database 170 from running such operations.

On the database 170, each user profile 175 (sometimes herein referred to as a user account, user information, or subject profile) can store and maintain information related to a user of the application 125 through user device 110. Each user profile 175 may be associated with or correspond to a respective user of the application 125. The user profile 175 may identify various information about the user, such as a user identifier, the condition to be addressed, information on sessions conducted by the user (e.g., activities or lessons completed), message preferences, user trait information, and a state of progress (e.g., completion of endpoints) in addressing the condition, among others. The information on a session may include various parameters of previous sessions performed by the user and may be initially null. The message preferences can include treatment preferences and user input preferences, such as types of messages or timing of messages preferred. The message preferences can also include preferences determined by the session management service 105, such as a type of message the user may respond to. The progress may initially be set to a start value (e.g., null or "0") and may correspond to alleviation, relief, or treatment of the condition. The user profile 175 may be continuously updated by the application 125 and the session management service 105.

In some embodiments, the user profile 175 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 175 can include at least one activity log of messages provided to the user, interactions by the user identifying performance of the specific user, and responses from the user device 110 associated with the user, among others. The user profile 175 may be stored and maintained in the database 170 using one or more files (e.g., extensible markup language (XML), comma-separated values (CSV) delimited text files, or a structured query language (SQL) file). The user profile 175 may be iteratively updated as the user performs additional sessions or responds to additional messages.

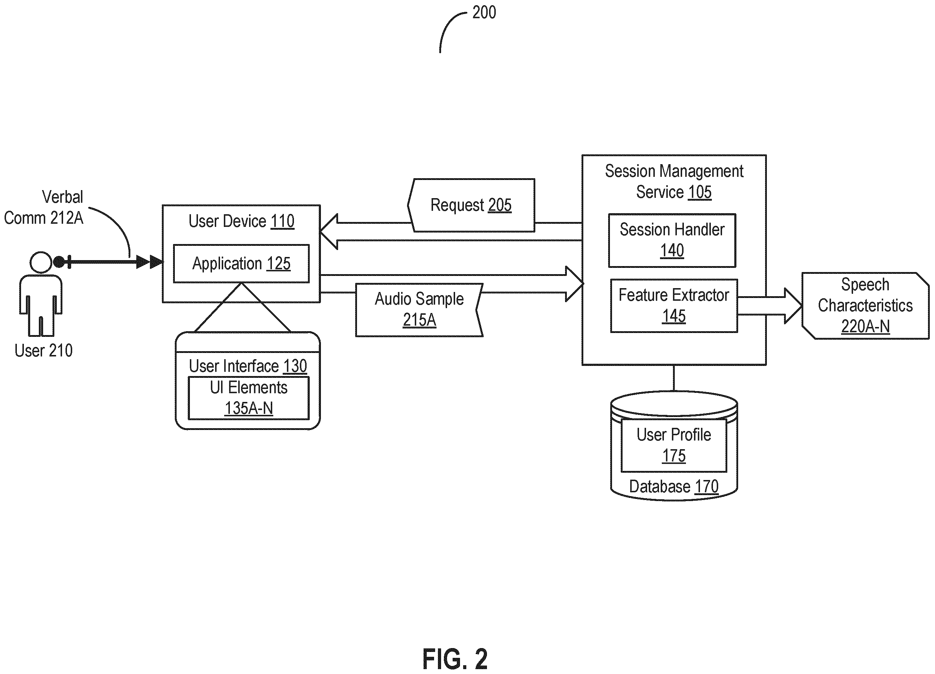

Referring now to FIG. 2, depicted is a block diagram of a process 200 of parsing audio samples in the system 100 for providing instructions. The process may include or correspond to operations performed by the system 100 to parse audio samples. Under process 200, the session handler 140 executing on the session management service 105 may send or transmit a request 205 to the application 125. The session handler 140 may generate the request 205 based on the user profile 175. The request 205 may take various formats associated with the application 125. For instance, the request 205 may appear as a notification directly on the application 125. In another instance, the request 205 may appear as an electronic communication (e.g., email) on the user 210. In another instance, the request 205 may appear as a short message service (SMS) (e.g., text message) or multimedia message service (MMS) (e.g., audio message, video message).

The request 205 may include a message with directions for a user 210 of the application 125 via the user device 110. The directions may give next steps to progress through the treatment plan based on the user profile 175. For example, the directions may indicate actions to be taken by the user 210 of the application 125 for the next stage of the treatment plan. The message of the request 205 may identify or include one or more words that the user 210 is to record via the application 125. The one or more words can be in the form of a set of text strings to be presented on the user interface 130 of the application 125. The session handler 140 can select or identify the one or more words to be provided via the request 205 based on the user profile 175. In some embodiments, the session handler 140 can select the one or more words based on a treatment plan specified for the user 210 as defined in the user profile 175. In some embodiments, the request 205 may include or identify a context in which the one or more words are to be recorded via the application 125. The context may define an environmental setting in which the user 210 is to record the words. The context, for example, may include a role play scenario in which the user 210 is to imagine when speaking the words as directed by the application 125.

Upon receipt of the request 205, the application 125 on the user device 110 can display, render, or otherwise present the message of the request 205 via the user interface 130. The message may prompt the user 210 to record an utterance of the one or more specified words. The application 125 can obtain, acquire, or otherwise record at least one verbal communication 212A via a microphone on the user device 110. The verbal communication 212A can correspond to or include an utterance of the one or more words by the user 210. The utterance can correspond to an action by the user 210 to verbally or orally produce the one or more words. In order to record the verbal communication 212A from the user 210, the application 125 may include a button on the user interface 130 to a recording. For instance, the user 210 may hit "record" on the user interface 130 to trigger the application 125 to create a recording to capture the verbal communication 212A by the user 210.

From recording the verbal communication 212A, the application 125 can output, produce, or otherwise generate at least one audio sample 215A. The audio sample 215A may correspond to or include one or more audio files. The audio files can be generated in accordance with any number of audio formats, such as a waveform audio format (WAV), an audio interchange file format (AIFF), a moving picture experts group format (MPEG), or an Ogg Vorbis (Ogg), among others. With the acquisition of the audio sample 215A, the application 125 can provide, transmit, or otherwise send the audio sample 215A to the session management service 105.

In some embodiments, the application 125 can obtain, acquire, or otherwise record at least one non-verbal communication from the user 210 via a camera of the user device 110, at least in partial concurrence with the verbal communication 212A. The non-verbal communication can correspond to or include actions by the user 210 while uttering the one or more words for the verbal communication 212A, such as gesturing, facial expression, or eye contact. From recording the non-verbal communication, the application 125 can output, produce, or otherwise generate at least one video sample (or an image). The video sample can be generated in accordance with any number of video formats, such as a moving picture experts group format (MPEG), a Windows Media Video (WMV), a QuickTime Movie (MOV), or Audio Video Interleave (AVI), among others. The non-verbal communication can be used to augment the verbal communication 212A in assessing the utterance by the user 210. Other input/output (I/O) devices of the user device 110 can be used to record the non-verbal communication.

The session handler 140 can retrieve, receive, or identify the audio sample 215A of the verbal communication 212A from the user 210 sent by the user device 110. In some embodiments, the session handler 140 can retrieve, receive, or identify the video sample of the non-verbal communication. The session handler 140 can identify the audio sample 215A (and the video sample) as associated with the one or more words selected for the user 210. The session handler 140 can store and maintain the audio sample 215A (and the video sample) on the database 170. The audio sample 215A can be stored as associated with the user profile 175.

The feature extractor 145 executing on the session management service 105 can process and parse the audio sample 215A. From the parsed audio sample 215A, the feature extractor 145 can extract, determine, or otherwise generate a set of acoustic features. For example, the feature extractor 145 may extract information or data from the audio sample 215 during the parsing process. The set of acoustic features may include spectral characteristics, temporal patterns, or pitch information, among others. The temporal patterns may include, for example, zero crossing rate, root mean square (RMS) energy, and temporal centroid, among others. The spectral characteristics may include, for example, Mel-frequency cepstral coefficients, spectral centroid, spectral flux, and spectral roll-off, among others. The set of acoustic features can be determined using a number of speech processing algorithms, such as speech recognition algorithms, a formant analysis algorithm (e.g., linear predictive coding), or a Mel-frequency cepstral coefficients (MFCC) extraction algorithm, among others.

The feature extractor 145 may generate, identify, or otherwise determine a set of speech characteristics 220A-N (hereinafter generally referred to as speech characteristics 220) using the parsed audio sample 215A. The speech characteristics 220 can define or identify various aspects of the verbal communication 212A of the user 210 determined from the audio sample 215A. In some embodiments, the feature extractor 145 can determine the set of speech characteristics 220 using the audio sample 215A in accordance with any number of speech processing algorithms. The audio sample 215A itself and the one or more words specified to be uttered by the user 210 may be used to determine the set of speech characteristics 220. The algorithm may include, for example, a speech recognition algorithm (e.g., a deep learning algorithm), a pitch estimation algorithm, a speech rate measurement algorithm, a prosody detection algorithm, a voice quality analysis (e.g., jitter and shimmer analysis), or a rhythm pattern recognition, among others.

The set of speech characteristics 220 may identify or include respiration, phonation, articulation, resonance, prosody, pitch, jitter, shimmer, rhythm, pacing, or pausing, among others. The respiration may correspond to a duration of the duration of inspiration of the verbal communication 212A. The phonation may correspond to a vocal fold oscillation in uttering the one or more words for the verbal communication 212A. The articulation may correspond to clarity and precision with which speech sounds are produced in the verbal communication 212A. The resonance may correspond to vibration of sound waves in the oral and nasal cavities in the verbal communication 212A. The prosody may correspond to variations in pitch, duration, and intensity in the utterance of one or more words. The pitch may correspond to a frequency (or highness or lowness) in the utterance of one or more words of the verbal communication 212A. The jitter may correspond to frequency variation (or pitch) in the verbal communication 212A. The shimmer may correspond to a measure of the amplitude or intensity variation. The rhythm may correspond to a pattern and timing of speech sounds and pauses. The pacing may correspond to a rate or speed at which speech is produced in the verbal communication 212A. The pausing may correspond to breaks or silences during the utterance. In some embodiments, the speech characteristics 220 may include or identify a number of words in the verbal communication 212A and a length (e.g., a time duration) of the words in the verbal communication 212A.

For each speech characteristic 220, the feature extractor 145 may calculate, generate, or otherwise determine a score for the speech characteristic 220. Each score can be identified or defined along a scale for the corresponding speech characteristic 220. In some embodiments, the score may be generated based on a degree of severity of one or more speech characteristics 220A-N. The degree of severity may be calculated based on a deviation from a normal speech characteristic. The normal speech characteristic may be stored in the database 170 and the feature extractor 145 may compare the generated speech characteristics 220 and the normal speech characteristics 220. For example, a normal speech rate is 150 words per minute, but a user 210 may have a speech rate of 100 words per minute. The deviation may be assigned a value of 50 to indicate the user is 50 words away from the normal speech characteristics 220. In another example, a normal speech rhythm is three to eight syllables per second, but a user 210 may have a speech rhythm that is one syllable per second. The deviation may be assigned a value of two to indicate the user is two syllables away from the normal speech characteristics 220. In some embodiments, the normal speech characteristic may depend on the context (e.g., role-play scenario) provided in the request 205 to the user 210. For instance, the normal speech characteristic may specify that the expected number of words is 100-250 words over a 1 to 2 minute time window for a scenario on asking directions to a bystander.

In some embodiments, the feature extractor 145 may process and parse the video sample of the non-verbal communication from the user 210. The feature extractor 145 may generate, identify, or otherwise determine a set of non-verbal characteristics using the video sample. The set of non-verbal characteristics may include, for example, a gesture (e.g., hand gesture, body pose, or head movement), a facial expression demonstrating an emotion (e.g., happiness, sadness, anger, fear, surprise, or contempt), or eye contact (e.g., eye gaze), among others. The feature extractor 145 may use any number of algorithms to extract the set of the non-verbal characteristics. The algorithms may include, for example, a computer vision algorithm (e.g., a deep learning artificial neural network, a dynamic time warping (DTM), or a scale-invariant feature transform (SIFT)) or an eye gaze algorithm (e.g., eye tracking algorithm, a pupil detection algorithm, iris-based method, or gaze estimation), among others. In some embodiments, the feature extractor 145 may determine a score for each non-verbal characteristic. Each score can be identified or defined along a scale for the corresponding non-verbal characteristic.

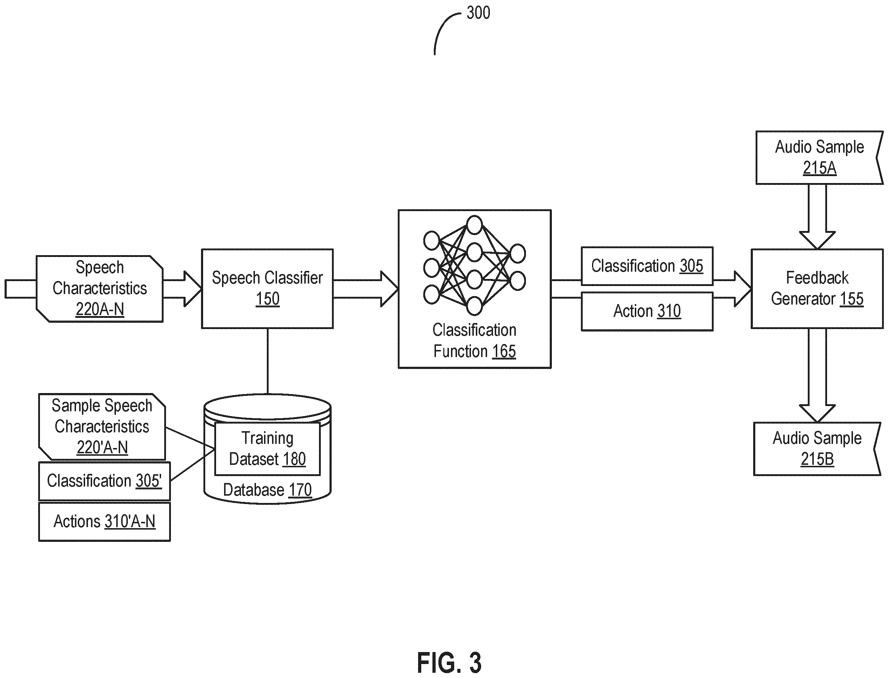

Referring now to FIG. 3, depicted is a block diagram of a process 300 of applying classification of speech in the system 100 for providing instructions. The process may include or correspond to operations performed by the system 100 to apply classification of speech in the system 100 for providing instructions. Under process 300, the speech classifier 150 executing on the session management service 105 may generate, identify, or otherwise determine at least one speech classification 305 from a set of speech classifications based on the set of speech characteristics 220. In some embodiments, the speech classifier 150 may determine the classification 305 based on the set of non-verbal characteristics, along with the set of speech characteristics 220. The set of speech classifications may include, for example, mumbling, lisp, dysarthria, stuttering, or understandable (or intelligible), among others. Mumbling may correspond to speaking in a low, indistinct, or unclear manner, with insufficient articulation or pronunciation. Lisping may correspond to mispronunciation of certain sounds (e.g., sibilant speech such as 's' or 'z'). Dysarthria may correspond to slurred, slow, or imprecise speech. Stuttering may correspond to repetitions of sounds, syllables, or words, prolongations of sounds, or involuntary pauses. Understandable (or intelligible) may correspond to speech that is comprehendible by others, and may lack other defects such as mumbling, lisping, dysarthria, or stuttering.

The speech classifier 150 can determine the speech classification 305 in accordance with the classification function 165. The classification function 165 may be used to identify or determine the speech classification 305 based on the speech characteristics 220 and the non-verbal characteristics. The classification function 165 may include, for example, an average of the scores corresponding to the speech characteristics 220 (and non-verbal characteristics); a weighted combination of scores corresponding to the speech characteristics 220; a mapping of scores to different speech classifications; a machine learning model; and a generative transformer model, among others. In some embodiments, the speech classifier 150 can determine an average of scores corresponding to the speech characteristics 220 (and non-verbal characteristics). The classification function 165 may define a mapping between average scores and speech classifications. Using the average, the speech classifier 150 can identify the speech classification 305 as defined by the classification function 165.

In some embodiments, the speech classifier 150 may determine the classification 305 using the weighted combination of scores corresponding to the speech characteristics 220 (and non-verbal characteristics). The classification function 165 may define the weights for each score and a mapping between the weighted values and one of the set of speech classifications. Based on the weighted combination, the speech classifier 150 may identify the speech classification as defined by the classification function 165. For example, a user 210 may have a respiration characteristic, articulation characteristic, and a pitch characteristic with scores of 1, 26, and 79 respectively. The speech classifier 150 may calculate a weighted combination of the scores and may determine the classification 305 as lisp based on the weighted combination and the mapping defined by the classification function 165. In some embodiments, the speech classifier 150 may determine the classification 305 based on comparison between the set of speech characteristics 220 with the mapping of scores to different speech classifications as defined by the classification function 165. In some embodiments, the speech classifier 150 may determine the classification 305 based on the number of words spoken and the length of the duration of speech as identified in the speech characteristics 220.

In some embodiments, the speech classifier 150 may apply the set of speech characteristics 220 (and the non-verbal characteristics) to the machine learning model of the classification function 165 to determine the speech classification 305. To apply the set of speech characteristics 220, the speech classifier 150 may feed the set of speech characteristics 220 as inputs into the machine learning model. Upon feeding the set of speech characteristics 220 into the machine learning model, the speech classifier 150 may process the input in accordance with the set of weights defined by the machine learning model, and generate the speech classification 305 as an output of the machine learning model. In some embodiments, the speech classifier 150 may apply the set of speech characteristics 220 (and the non-verbal characteristics) to the generative transformer of the classification function 165 to determine the speech classification 305. To apply the set of speech characteristics 220, the speech classifier 150 may generate an input prompt using the set of speech characteristics 220 in accordance with a template. The speech classifier 150 can feed the prompt into the generative transformer model. Upon feeding the prompt into the generative transformer model, the speech classifier 150 may process the input in accordance with the set of weights defined by the generative transformer model, and generate the speech classification 305 as an output of the generative transformer model.

The machine learning model (or the transformative model) of the classification function 165 may be initialized, established, and trained using the training dataset 180 on the database 170. The training dataset may include a set of examples. Each example may include a set of sample speech characteristics 220'A-N (hereinafter generally referred to as characteristics 220'), an expected classification 305', and an expected one or more actions 310' A-N (hereinafter generally referred to as actions 310'). In some embodiments, each example may include a sample audio recording from which the sample speech characteristics 220' are derived. To train, the speech classifier 150 (or another computing device) may apply the sample speech characteristics 220' to the machine learning model of the classification function 165 to generate a predicted classification and action. The speech classifier 150 may compare the output predicted classification and action from the machine learning model with the expected classification 305' and actions 310' of the example in the training dataset 180. Based on the comparison, the speech classifier 150 may determine a loss metric in accordance with a loss function (e.g., a mean squared error, cross-entropy loss, hinge loss, or Huber loss). Using the loss metric, the speech classifier 150 can update the one or more metrics of the machine learning model of the classification function 165.

Based on the speech classification 305, the speech classifier 150 may identify or select at least one action 310 from a set of candidate actions. The action 310 may indicate or identify a modification to the one or more of the speech characteristics 220 to define an utterance for the user 210. The set of actions 310 may identify or include, for example, an alteration in any of the speech characteristics 220, such as respiration, phonation, articulation, resonance, prosody, pitch, jitter, shimmer, rhythm, pacing, or pausing, among others, in the utterance of the words by the user 210. When the speech classification 305 indicates that the verbal communication 212A is able to be understood, the speech classifier 150 may select the action 310 for the user 210 to maintain one or more of the set of speech characteristics 220 for the utterance. Conversely, when the speech classification 305 indicates that the verbal communication 212A is not able to be understood, the speech classifier 150 may select the action 310 for the user 210 to modify one or more of the set of speech characteristics 220 for the utterance. In some embodiments, the set of actions 310 may identify or include a target number (or range) of words or a target length (e.g., time duration) of the utterance.

In some embodiments, the speech classifier 150 may select the action 310 based on the set of speech characteristics 220 (and the non-verbal characteristics). For example, if the user 210 is determined to have prosody issues, the action 310 may specify a variation in pitch during the course of the utterance of the one or more words. The selection of the action 310 may be based on a defined mapping between scores of speech characteristics 220 to different candidate actions. In some embodiments, the speech classifier 150 may use the classification function 165 to determine the action 310. For instance, the speech classifier 150 may apply the set of speech characteristics 220 to the machine learning model of the classification function 165. By processing the input speech characteristics 220 in accordance with the weights of the model, the speech classifier 150 may generate the action 310 to be taken by the user 210.

In some embodiments, the speech classifier 150 may select or identify at least one factor as a cause of the speech classification 305 from a set of candidate factors based on the set of speech characteristics 220. The candidate factors may identify potential causes contributing to the speech classification 305. The set of candidate factors may include speech characteristics, such as respiration, phonation, articulation, resonance, prosody, pitch, jitter, shimmer, rhythm, pacing, or pausing, among others. To identify, the speech classifier 150 may compare the score for each speech characteristic 220 with an expected range of values for the speech characteristic for understandable speech. If the score is outside the expected range, the speech classifier 150 may select the speech characteristic as a factor. Otherwise, if the score is within the expected range, the speech classifier 150 may exclude the speech characteristic as a factor. For instance, the speech classifier 150 may determine that the score for articulation is lower compared to a reference range, and may identify articulation as the factor causing the mumbling as identified for the speech classification 305.

The feedback generator 155 executing on the session management service 105 can write, output, or otherwise generate an audio sample 215B for playback for the user 210. The audio sample 215B may be an altered or modified version of the original audio sample 215A in accordance with the action 310. In some embodiments, the feedback generator 155 can use the one or more words specified to be uttered by the user 210 along with the original audio sample 215A and the action 310 in generating the audio sample 215B. To generate the second audio sample 215B, the feedback generator 155 can change, alter, or otherwise modify the set of acoustic features extracted from the original audio sample 215A based on the action 310 and the words. For example, if the speech in the original audio sample 215A is characterized as having a lisp, the feedback generator 155 may insert frequency components within the audio sample 215B to add sibilant consonants (corresponding to words with such consonants) thereby removing instances of the lisp within the speech. The feedback generator 155 can use any number of speech or audio processing algorithms, such as a text-to-speech (TTS) algorithm, a pitch shifting algorithm, a voice conversion model, a Formant modification algorithm, or a spectral envelope modification, among others.

In some embodiments, the feedback generator 155 may apply a speech synthesis model on the audio sample 215A and the action 310. The speech synthesis model may include any number of machine learning models, such as a deep learning speech synthesis model or a generative transformer model, among others. The speech synthesis model may include a text analyzer to convert text into linguistic features, an acoustic model to extract features from the original recording based on the linguistic features, and a vocoder to create the waveform for the corrected speech, among others. The speech synthesis model may be trained using a training dataset include a set of examples. Each example may include a sample audio recording, one or more words used in the utterance of the sample audio recording, an action (e.g., the action 310) to be applied, and an expected output from applying the action to the sample audio recording. The feedback generator 155 may feed the audio sample 215A, the one or more words, and the action 310 to the speech synthesis model. Upon feeding, the feedback generator 155 may process the input audio sample 215A and the action 310 according to the set of weights of the speech synthesis model. From processing, the feedback generator 155 may generate the corrected audio sample 215B.

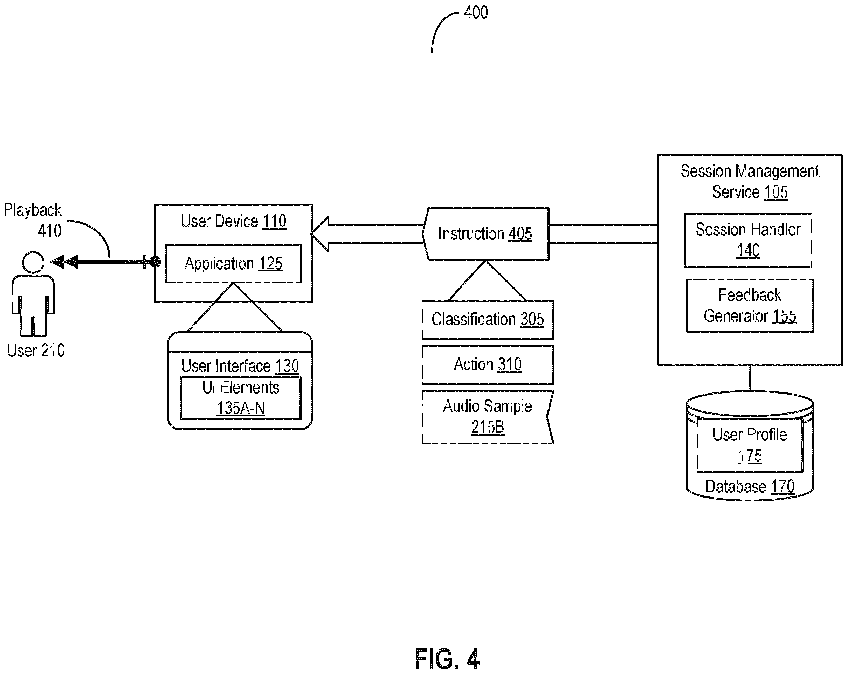

Referring now to FIG. 4, depicted is a block diagram of a process 400 of providing feedback in the system 100 for providing instructions. The process 400 may include or correspond to operations performed by the system 100 to providing feedback in the system 100 for providing instructions 405. Under process 400, the feedback generator 155 may write, output, or otherwise generate an instruction 405 for the user 210. The instruction 405 may include a message to prompt the user 210 to perform the utterance defined by the action 310. In some embodiments, the feedback generator 155 may generate the instruction 405 to include the message to identify the classification 305. In some embodiments, the feedback generator 155 may generate the instruction 405 to include the message to identify any one or more of: the speech characteristics 220 (e.g., including the scores), the speech classification 305, and the action 310 to be taken by the user 210, among others.

In some embodiments, the feedback generator 155 may generate the instruction 405 to include the message to identify the factor as the cause for the speech classification 305, among others. In some embodiments, the feedback generator 155 may generate the instruction 405 to include the altered audio sample 205B. In some embodiments, the feedback generator 155 may generate a directive for the user 210 to perform to include the instruction 405. For example, the directive may include, "For the next three days, please conduct a pursed lip exercise every eight hours." With the generation of the instruction 405, the session handler 140 may send, transmit, or otherwise provide the instruction 405 to the user device 110.

Upon receipt of the instruction 405, the application 125 on the user device 110 may render, display, or otherwise present the message of the instruction 405. The message may be to prompt the user 210 to perform the utterance defined by the action 310. The message may identify any one or more of the set of speech characteristics 220 (including the scores), the classification 305, the action 310, and the factor, among others, via the UI elements 135 of the user interface 130. In some embodiments, the application 125 may present a playback 410 of the audio sample 215B. The playback 410 may be in a video or as audio. In some embodiments, the application 125 may present the action 310 as tutorial videos, practice sessions, interactive exercises, animations, and guided practices among others. For example, a sip and say exercise may be presented on the application 125 in a video with a speech pathologist to guide the exercise. In another example, animations of the shape of an individual's lips may be placed on a portion of the application 125 using the UI elements 135.

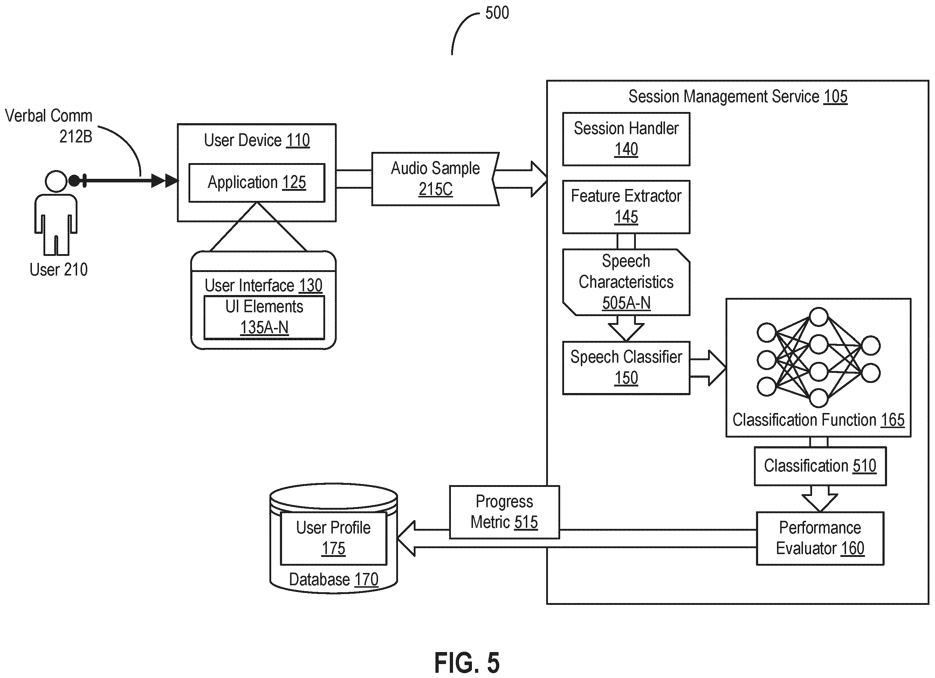

Referring now to FIG. 5, depicted is a block diagram of a process 500 of tracking progress of users 210 in the system for providing instructions. The process 500 may include or correspond to operations performed by the system 100 to track progress of users 210. The process 500 may include any number of operations of processes 200, 300, and 400. Under process 200, the session handler 140 may start a subsequent session by sending a request. The request may include a message with directions for a user 210 of the application 125 via the user device 110. The message of the request 205 may identify or include one or more words that the user 210 is to record via the application 125. The one or more words may be different from the one or more words that the user 210 was instructed to utter in a previous session.

The application 125 on the user device 110 can display, render, or otherwise present the message of the request 205 via the user interface 130. The message may prompt the user 210 to record an utterance of the one or more specified words. The application 125 can obtain, acquire, or otherwise record at least one verbal communication 212A via a microphone on the user device 110. The verbal communication 212B can correspond to or include an utterance of the one or more words by the user 210. The utterance can correspond to an action by the user 210 to verbally or orally produce the one or more words. From recording the verbal communication 212B, the application 125 can output, produce, or otherwise generate at least one audio sample 215C. With the acquisition of the audio sample 215C, the application 125 can provide, transmit, or otherwise send the audio sample 215C to the session management service 105. In some embodiments, the application 125 can obtain, acquire, or otherwise record at least one non-verbal communication from the user 210, at least in partial concurrence with the verbal communication 212C via a camera of the use device 110.

The session handler 140 may receive the audio sample 215C (and the video sample) from the user device 110. Upon receipt, the feature extractor 145 may generate, identify, or otherwise determine a set of speech characteristics 505A-N (hereinafter generally referred to as characteristics 505) using the parsed audio sample 215C. The speech characteristics 505 can be determined in a similar manner as the speech characteristics 220. The speech characteristics 505 can define or identify various aspects of the verbal communication 212B of the user 210 determined from the audio sample 215C. For each set of speech characteristic 220, the feature extractor 145 may calculate, generate, or otherwise determine a score for the speech characteristic 505. Each score can be identified or defined along a scale for the corresponding speech characteristic 505. The feature extractor 145 may generate, identify, or otherwise determine a set of non-verbal characteristics using the video sample. In some embodiments, the feature extractor 145 may determine a score for each non-verbal characteristic. Each score can be identified or defined along a scale for the corresponding non-verbal characteristic.

The speech classifier 150 may generate, identify, or otherwise determine at least one speech classification 510 from a set of speech classifications based on the set of speech characteristics 505. The speech classification 510 can be determined in a similar manner as the speech classification 305. In some embodiments, the speech classifier 150 may determine the classification 510 based on the set of non-verbal characteristics, along with the set of speech characteristics 505. In some embodiments, the speech classifier 150 can determine the speech classification 505 in accordance with the classification function 165. Based on the speech classification 505, the speech classifier 150 may identify or select at least one action from a set of candidate actions. The action may indicate or identify a modification to the one or more of the speech characteristics 505 to define an utterance for the user 210.

The performance evaluator 160 executing on the session management service 105 may calculate, determine, or otherwise generate at least one progress metric 515 based on a comparison between the classification 510 of the current verbal communication 212B and the classification 305 from a prior verbal communication 212A. In some embodiments, the performance evaluator 160 may determine the progress metric 515 based on a comparison between the speech characteristics 505 of the current verbal communication 212B and the speech characteristics 220 from a prior verbal communication 212A. The progress metric 515 may correspond to or identify a quantification of the improvement or deterioration between the current verbal communication 212B and one or more prior verbal communications (e.g., the verbal communication 212A). For example, a low progress metric 515 for classification 510 may indication a low deviation from classification 305, indicating slow improvement. In another example, a high progress metric 515 for classification 510 may indicate a high deviation from classification 305, indicating rapid improvement. In some embodiments, the progress metric 515 may generate a function of a level of deviation between classification 305 and classification 510. The function may include percentage improvement, relative improvement, improvement ratio, or an improvement index. The level of deviation can indicate how fast or slow the user 210 is improving based on the execution of the instructions 405. With the determination of the progress metric 515, the performance evaluator 160 may store and maintain the progress metric 515 in the user profile 175 of the user 210.

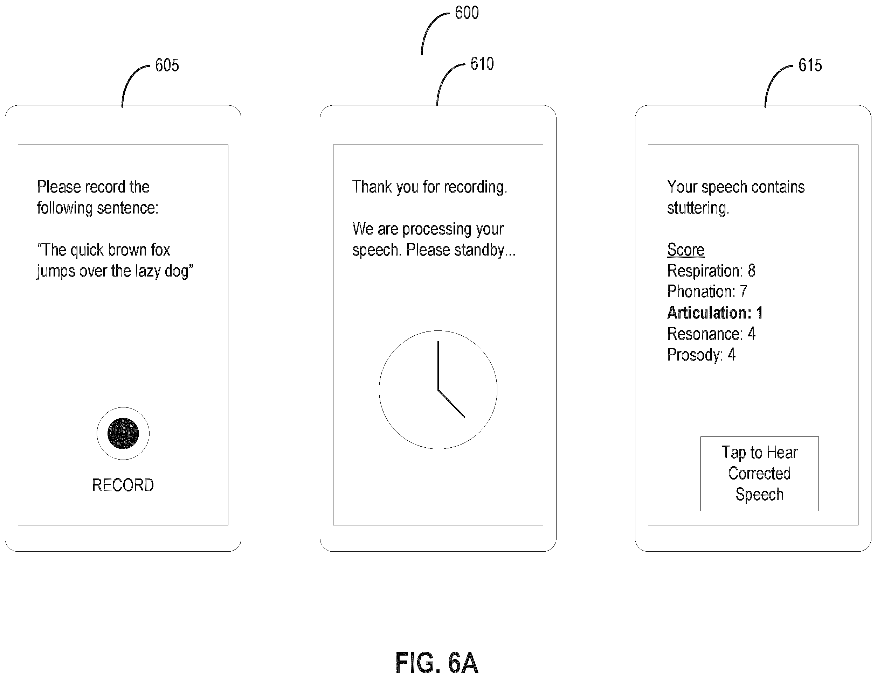

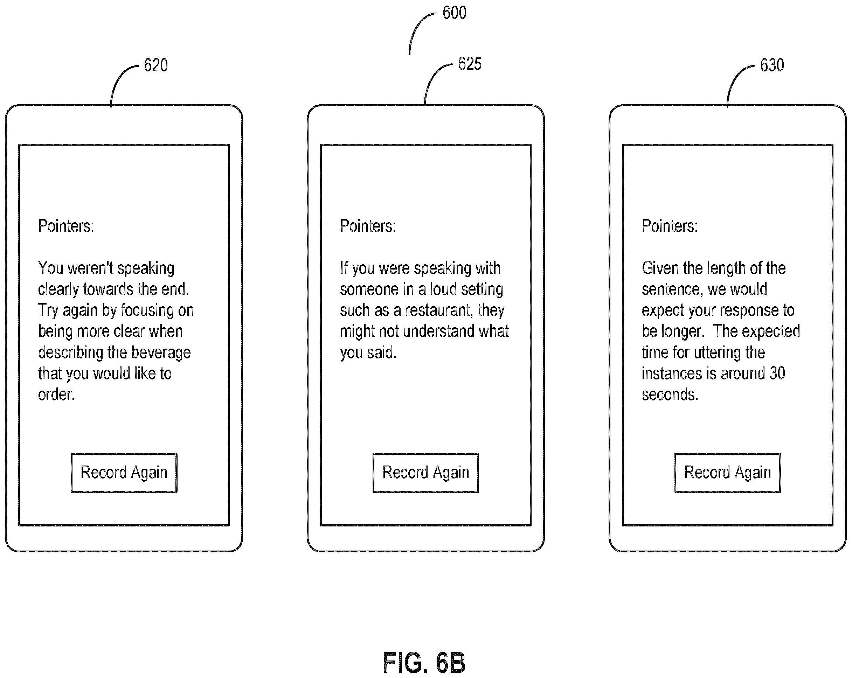

Referring now to FIG 6A and 6B, depicted are screenshots of a set 600 of user interfaces in the system for providing instructions for speech based on speech classifications of verbal communications from users. The user interfaces in the 600 may be part of the application 125, and presented through the user interface 130. The user interface 605 may be a prompt to the user to record a sentence. The user interface 610 may be a wait screen as the audio sample from the user is processed and analyzed. The user interface 615 can display a classification of a speech defect (e.g., mumbling), along with scores for various speech characteristics (e.g., respiration, phonation, articulation, resonance, and prosody) from the user's speech sample. The user interface 615 may also include a button for the user to press to listen to a playback of corrected audio speech. In addition, the user interface 620 can provide feedback in the form of text, for example, to direct the user to improve clarity, and can include a button to record the speech sample again. The user interface 625 can provide feedback with context, notifying the user that the user's speech might not be suitable in certain settings, and can include a button to record the speech sample again. The user interface 630 can textual feedback informing the user that the length of the recorded sentence is short of the expected time, and can include a button to record the speech sample again.

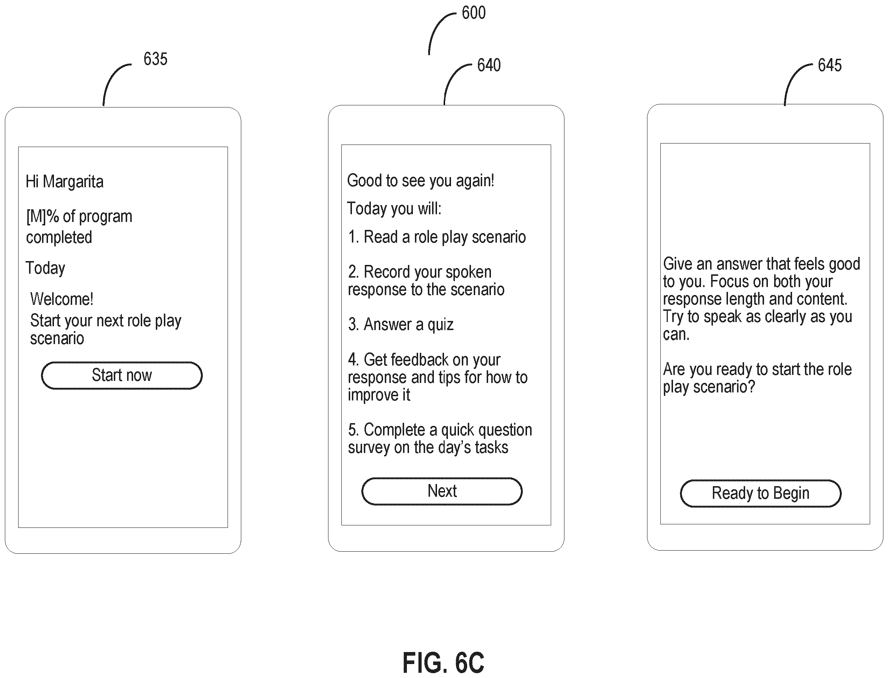

Referring now to FIG. 6C, depicted are screenshots of an additional set of user interfaces 600 in the system for providing instructions for speech based on speech classifications of verbal communications from users. The user interfaces 635-645 may be part of role play practice sessions. The sessions may include, for example, asking if a seat is taken at the bus, among others. The user interface 635 may indicate a percentage of the sessions completed by the user. The user interfaces 640 and 645 may be an introductory screen to a role play practice session.

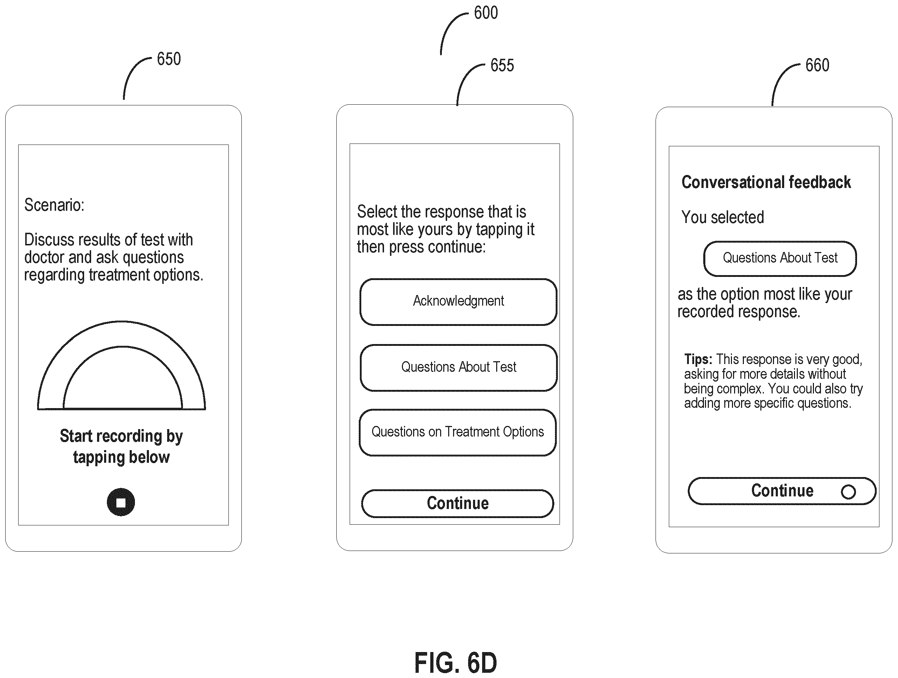

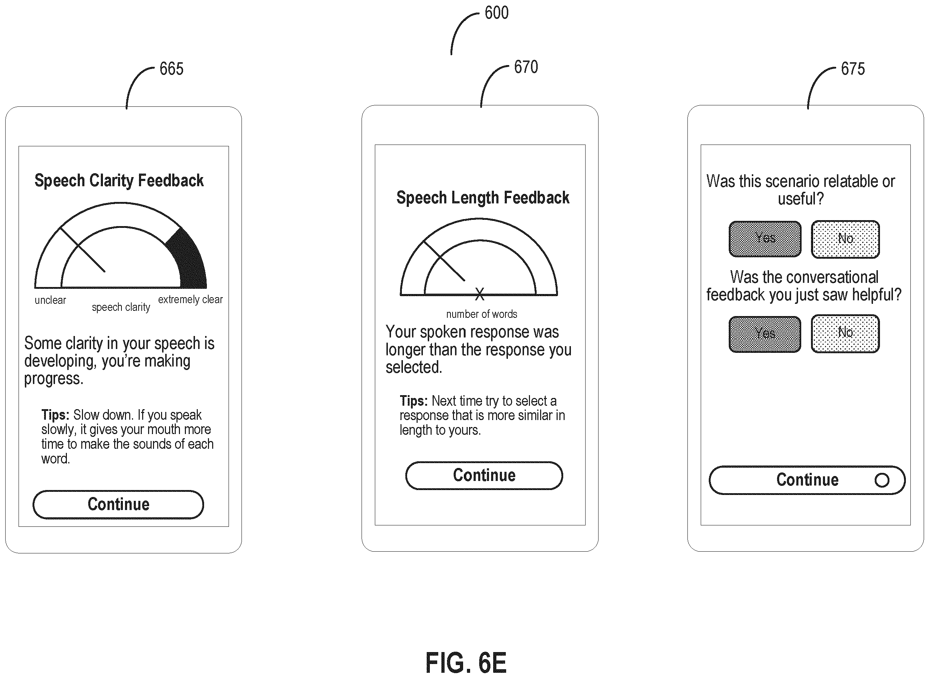

Referring now to FIGs. 6D and 6E, depicted are screenshots of an additional set of user interfaces 600 in the system for providing instructions for speech based on speech classifications of verbal communications from users. The user interface 650 may be to prompt the user to record given a role-play scenario (e.g., speaking with a doctor). Users were provided with one role-play scenario. Users can record one audio recording per session and were provided feedback about the response length during the recording using the dial indicator. The user interface 655 may be to direct the user to select the response most similar to theirs. The user interface 660 may indicate the response selected by the user. The user interface 665 may provide feedback regarding the clarity of the user's speech in the audio recording. The user interface 660 may provide feedback regarding speech length of the user's speech in the audio recording. The user interface 675 may prompt the user to select a usefulness of the feedback to the application.

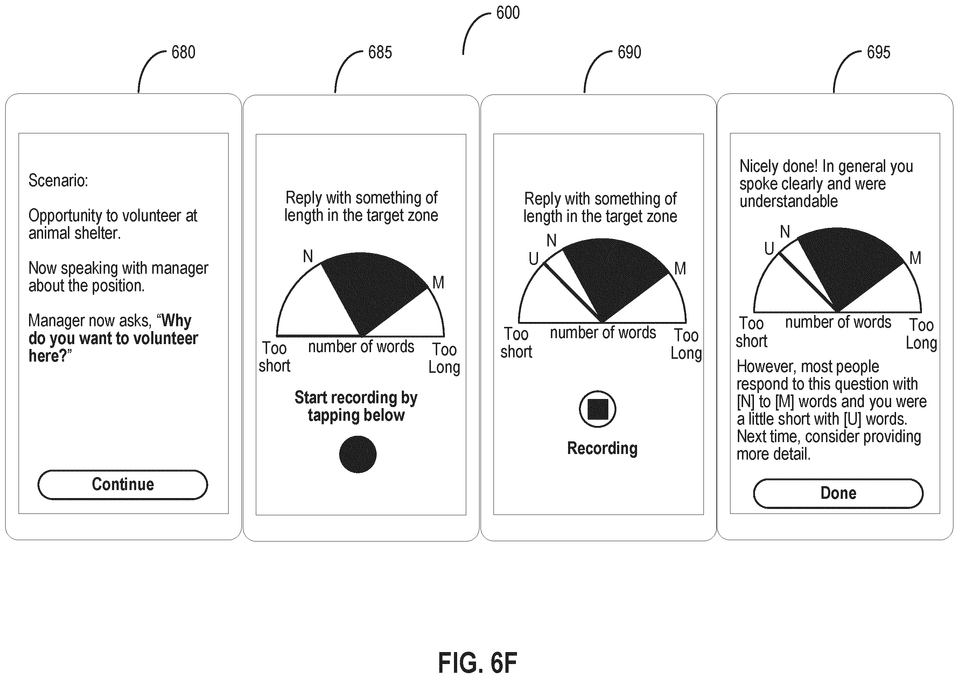

Referring now to FIG. 6F, depicted are screenshots of an additional set of user interfaces 600 in the system for providing instructions for speech based on speech classifications of verbal communications from users. The user interface 680 may prompt the user to envision themselves in a given role-play scenario (e.g., speaking with a volunteer manager). Upon interacting with the "continue" button, the user interface 685 may be displayed and may prompt the user to record a conversation with a target range for the number of words in the recording. With the finishing of the recording, the user interface 690 may be presented and may provide feedback indicating the number of words spoken by the user. The user interface 695 may include additional feedback in the form of text indicating that the user should have spoken an additional number of words.

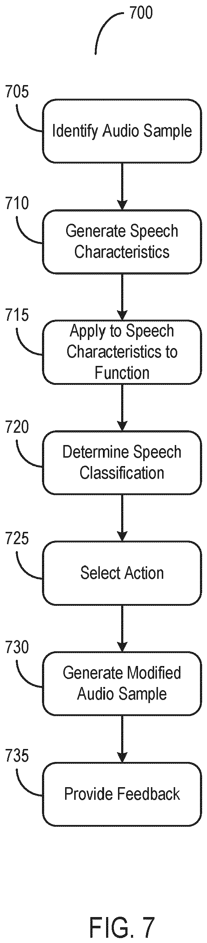

Referring now to FIG. 7, depicted is a flow diagram of a method 700 of providing instructions for speech based on speech classifications of verbal communications from users. The method 700 may be implemented or performed using any of the components detailed herein, such as the session management service 105 and the user device 110, or any combination thereof. Under the method 700, a computing system (e.g., the session management service 105 or the user device 110) may identify an audio sample (705). The computer may generate a set of speech characteristics (710). The computer may apply the set of speech characteristics to a classification function (715). The computer may determine a speech classification from applying the classification function (720). The computer may select an action based on the speech classification (725). The computer may generate a modified audio sample using the action (730). The computer may provide feedback (735).

### B. Method of Ameliorating Defect of Speech Expressiveness in Users in Need Thereof

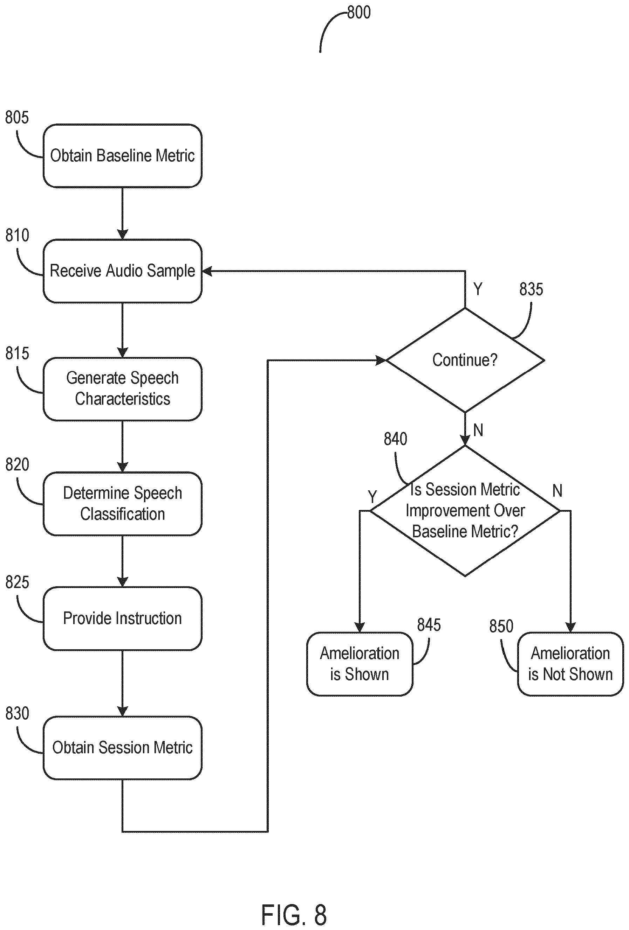

Referring now to FIG. 8, depicted is a flow diagram of a method 800 of ameliorating defect of speech expressiveness in a user in need thereof. The method 800 may be performed by any components or actors described herein, such as the session management service 105, the user device 110, or the user 210, among others. The method 800 may be used in conjunction with any of the functionalities or actions described herein in Section A. In brief overview, the method 800 may include obtaining a baseline metric (805). The method 800 may include receiving an audio sample (810). The method 800 may include generating speech characteristics (815). The method 800 may include determining an action (820). The method 800 may include providing an instruction (825). The method 800 may include obtaining a session metric (830). The method 800 may include determining whether to continue (835). The method 800 may include determining whether the session metric is an improvement over the baseline metric (840). The method 800 may include determining that amelioration is shown when the session metric is determined to be an improvement over the baseline metric (845). The method 800 may include determining that amelioration is not shown when the session metric is determined to be not an improvement over the baseline metric (850).

In further detail, the method 800 may include retrieving, identifying, otherwise obtaining a baseline metric (805). The baseline metric may be associated with a user (e.g., the user 210) with a defect in speech expressiveness. The baseline metric may be obtained (e.g., by a computing system such as the session management service 105 or the user device 110 or both) prior to providing any of the sessions to the user via a digital therapeutics application (e.g., the application 125 described herein). The baseline metric may indicate a degree of severity of the speech expressiveness the user. The baseline measure may depend on the type of condition, and may include those detailed herein in Examples 1-10.

The defect of the speech expressiveness may be caused by alogia or blunted vocal affect. Alogia may correspond to a reduction in the variety of speech or the quality of speech produced by the user. For example, the user experiencing alogia may exhibit a decreased fluency of speech, limited elaboration in their responses, or a general lack of meaningful content in communication. Blunted vocal affect may correspond to a reduction in the range and intensity of emotional expression in a user's voice. For instance, a user with blunted vocal affect may sound monotonous, lacking the normal fluctuations in pitch, tone, and rhythm that typically accompany different emotions, among others.

The user may be of any demographic or trait, such as by age (e.g., an adult (above age of 18) or late adolescent (between ages of 18-24)) or gender (e.g., male, female, or non-binary), among others. In some embodiments, the user with the defect in speech expressiveness may be diagnosed with or at risk of a condition. The condition may include any number of disorders that cause the speech defect in the user. The condition may include, for example, a speech pathology, a neurological disorder (e.g., schizophrenia with positive or negative symptoms, mild cognitive impairment, autism spectrum disorder (ASD), a neurodegenerative disease (e.g., Alzheimer's disease, dementia, or Parkinson's disease), or multiple sclerosis), an affective disorder (e.g., major depressive disorder, anxiety disorder, bipolar disorder or a post-traumatic stress disorder (PTSD)), among others. The schizophrenia in the user further may be with positive symptoms including hallucinations or delusions, or with negative symptoms including a decrease in motivation or emotional expressions.

The user may be receiving a treatment, at least in partial concurrence with the at least one of the sessions to be provided to the user. The treatment may include at least one of a psychosocial intervention or a medication to address schizophrenia. At least in partial concurrence with one or more of the sessions, the user may be receiving treatment for schizophrenia. The treatment may include a psychosocial intervention or a medication to address schizophrenia. The psychosocial intervention may include, for example, psychoeducation, group therapy, cognitive-behavioral therapy (CBT), or early intervention for first-episode psychosis (FEP), among others. The medication may include, for example, a typical antipsychotic (e.g., haloperidol, chlorpromazine, fluphenazine, perphenazine, loxitane, thioridazine, or trifluoperazine) or an atypical antipsychotic (e.g., aripiprazole, risperidone, clozapine, quetiapine, olanzapine, ziprasidone, lurasidone, paliperidone, or iclepertin), among others. The treatment may increase the efficacy of the medication that the user is taking to address the condition.

The method 800 may include retrieving, identifying, or otherwise receiving an audio sample of a first verbal communication from a user (810). The computing system can obtain, acquire, or otherwise record at least one verbal communication (e.g., verbal communication 212A or 212B) via a microphone. The verbal communication can correspond to or include an utterance of the one or more words by the user. The utterance can correspond to an action by the user to verbally or orally produce the one or more words. In some embodiments, the computing system may obtain, acquire, or otherwise record at least one non-verbal communication from the user, at least in partial concurrence with the verbal communication, via a camera. The non-verbal communication can correspond to or include actions by the user while uttering the one or more words for the verbal communication, such as gesturing, facial expression, or eye contact.

The method 800 may include determining, identifying, or otherwise generating a set of speech characteristics for the verbal communication using the first audio sample (815). The computing system may determine the set of speech characteristics (e.g., the speech characteristics 220 or 505) based on the audio sample of the verbal communication. The set of speech characteristics may identify or include respiration, phonation, articulation, resonance, prosody, pitch, jitter, shimmer, rhythm, pacing, or pausing, among others. For each speech characteristic, the computing system may calculate, generate, or otherwise determine a score for the speech characteristic. Each score can be identified or defined along a scale for the corresponding speech characteristic. In some embodiments, the computing system may process and parse the video sample of the non-verbal communication. The computing system may generate, identify, or otherwise determine a set of non-verbal characteristics using the video sample. The set of non-verbal characteristics may identify or include, for example, a gesture (e.g., hand gesture, body pose, or head movement), a facial expression demonstrating an emotion (e.g., happiness, sadness, anger, fear, surprise, or contempt), or eye contact (e.g., eye gaze), among others.

The method 800 may include selecting, identifying, or otherwise determining an action to modify one or more of the speech characteristics to define an utterance for the user (820). The action (e.g., the action 310) may indicate or identify a modification to the one or more of the speech characteristics to define an utterance for the user. computing system may determine the action based on the set of speech characteristics. In some embodiments, the computing system may determine at least one speech classification from a set of speech classifications based on the set of speech characteristics (or non-verbal characteristics). The set of speech classifications may include, for example, mumbling, lisping, dysarthria, stuttering, or understandable, among others. The computing system may use a classification function (e.g., the classification function 165) to determine the speech classification.

The method 800 may include providing an instruction presenting a message to prompt the user to perform the utterance defined by the action selected from the plurality of actions (825). The computing system may write, output, or otherwise generate the instruction (e.g., the instruction 405) for the user. The instruction may include a message to prompt the user to perform the utterance defined by the action. In some embodiments, the computing system may generate the instruction to include the message to identify any one or more of: the speech characteristics (e.g., including the scores), the speech classification, and the action to be taken by the user, among others. In some embodiments, the computing system may generate the instruction to include the altered audio sample (e.g., the audio sample 205B) of the original audio recording. Upon provision, the message may be presented to the user.

The method 800 may include retrieving, identifying, otherwise obtaining a session metric (835). The session metric may be obtained (e.g., by the computing system) subsequent to providing at least one session to the user via the digital therapeutics application. The session measure may indicate a degree of severity of the speech expressiveness the user, after being provided at least one session. The session measure may depend on the type of condition, and may include those detailed herein in Examples 1-10. The session metric may be of the same type of metric or scale as the baseline metric.

The method 800 may include identifying or determining whether to continue (840). The determination may be based on the set length (e.g., days, weeks, or years) of the trial, a set number of time instances during which to perform one or more sessions, or a set number of sessions to be provided to the user. For example, the set number of time instances may range from 3 days to 6 months (e.g., with trial ending 6 months from start), relative to the obtaining of the baseline metric or the start of the initial session by the user. When the amount of time from the obtaining of the baseline metric exceeds the set length, the determination may be to stop providing additional tasks. In contrast, when an amount of time has not exceeded the set length, the determination may be to continue providing additional tasks and repeat from (810).

The method 800 may include identifying or determining whether the session metric is an improvement over the baseline metric (845). The improvement may correspond to an amelioration in the degree of severity in the defect of speech expressiveness occurs in the user. The improvement may be shown when the session metric is increased compared to the baseline metric by a first predetermined margin or when the session metric is decreased compared to the baseline metric by a second predetermined margin. For example, for certain types of metrics, an improvement will be shown by an increase in scores between the baseline and the session. For other types of metrics, an improvement will be shown by a decrease in scores between the baseline and the session.. The margin may also depend on the type of metric used and may in general correspond to the difference in value showing noticeable difference by the clinician or user, or showing a statistically significant result in the difference in the values between the baseline and session metrics.

The method 800 may include determining that amelioration is shown when the session metric is determined to be an improvement over the baseline metric (850). In some embodiments, the amelioration may be determined (e.g., by the computing system or a clinician examining the user) to occur when the session metric is increased from the baseline metric by the second predetermined margin. In some embodiments, the amelioration may be determined (e.g., by the computing system or a clinician examining the user) to occur when the session metric is decreased from the baseline metric by the first predetermined margin. The method 800 may include determining that amelioration is not shown when the session metric is determined to be not an improvement over the baseline metric (855). In some embodiments, the amelioration may be determined (e.g., by the computing system or a clinician examining the user) to not occur when the session metric is not increased from the baseline metric by the second predetermined margin. In some embodiments, the amelioration may be determined (e.g., by the computing system or a clinician examining the user) to not occur when the session metric is not decreased from the baseline metric by the second predetermined margin.

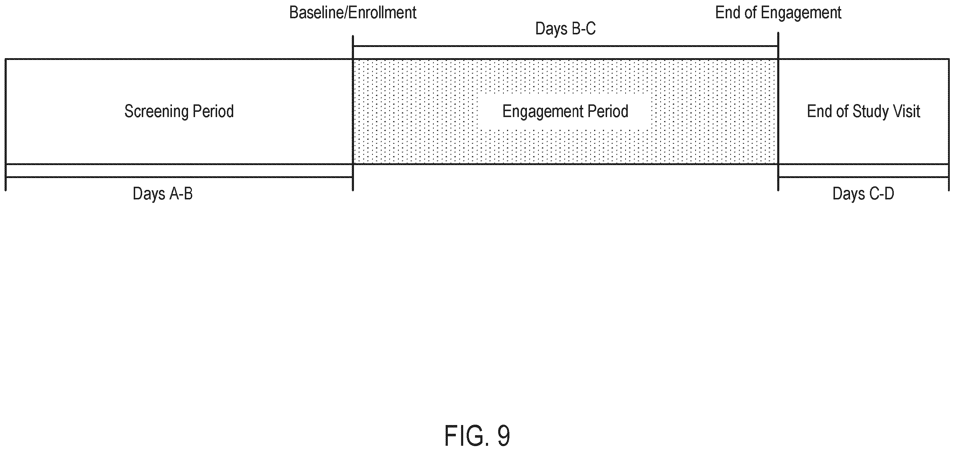

Referring now to FIG. 9, depicted is a block diagram of a study design for testing an application for ameliorating defects of speech expressiveness in users. In overview, during the screening visit, eligible participants downloaded and installed the application (e.g., the application 125) onto their user device. At the baseline visit, participants activated the application. During the engagement period, participants were asked to use the application daily (e.g., between 3 days and 6 months). In the follow-up visit, users uninstalled the application. There will be 10-300 participant (or users) in the study.

Screening Period (Day A to B) - All participants who have provided informed consent will enter a screening period (e.g., up to -7 to -21 days prior to engagement) to determine eligibility. Assessments and activities will be conducted. During the screening visit, site personnel will assist participants to download and install the application.

Baseline Visit (Day B) - During the baseline visit on Day B, participant eligibility will be confirmed. Assessments and activities will be conducted. Participants will be considered eligible to activate the application. Once enrolled, site personnel will assist participants to activate the application 125 and to complete a practice activity. The application content will only be available following activation at baseline.

Engagement Period (Day B to C) - Participants will enter an engagement period (e.g., ranging from 5 days to 6 months) during which they will interact with the application and complete assessments and activities.

End-of-Study Visit (Days C to D) - During the End-of-Study (EOS) visit between Days C to D (e.g., 7-14 days from end of engagement), participants will uninstall the application. Assessments and activities will be conducted.

### Example 1: Use of Application to Individuals with Speech Pathologies in General

In one example, the application (e.g., the application 125) will be given to individuals who have various speech pathologies per International Classification of Diseases 11th Revision (ICD-11) or Diagnostic and Statistical Manual of Mental Disorders, Fifth Edition (DSM-5). The speech pathologies can include, for example, articulation pathologies (e.g., articulation disorder, phonological disorder, childhood apraxia of speech, dysarthria, and speech sound disorder (SSD)), fluency pathologies (e.g., stuttering and cluttering), voice pathologies (e.g., spasmodic dysphonia, vocal cord paralysis, laryngitis, and dysphonia), resonance pathologies (e.g., hypernasality, hyponasality, and velopharyngeal dysfunction), or neurological speech pathologies (e.g., aphasia, apraxia of speech, and dysarthria), among others. The speech pathologies can be of mild-to-moderate impairment. The treatment will be 5 days to 6 months long. Improvements in defect in speech pathologies will be shown, as measured by a metric based on the speech classification (e.g., the classification 305) or any of the metrics in Examples 2-6, among others, over the course of using the application.

### Example 2: Use of Application to Individuals with Articulation Pathologies

In one example, the application (e.g., the application 125) will be given to individuals who have articulation pathologies per International Classification of Diseases 11th Revision (ICD-11) or Diagnostic and Statistical Manual of Mental Disorders, Fifth Edition (DSM-5). The articulation pathologies may include articulation disorder, phonological disorder, apraxia of speech, dysarthria, and speech sound disorder (SSD), among others. The speech pathologies can be of mild-to-moderate impairment. The treatment will be 5 days to 6 months long. Improvements in articulation disorder will be shown, as measured by Goldman-Fristoe Test of Articulation (GFTA-3), Arizona Articulation Proficiency Scale (Arizona-3), speech intelligibility index (SII), percentage of intelligible words (PIW), percent intelligible utterances (PIU), percentage of intelligible syllables (PIS), a percentage of consonants correct (PCC), percentage of vowels correct (PVC), percentage of vowels and diphthongs correct (PVC-R) or a metric based on speech classification (e.g., the classification 305), among others, over the course of using the application. Other metrics can be used to show improvements, such as those detailed herein in other Examples (e.g., Example 3-6).

Goldman-Fristoe Test of Articulation (GFTA-3) is an assessment tool used to evaluate an individual's ability to produce consonant sounds. The test is designed to assess sound production in various word positions (initial, medial, final). The test includes two main subtests: Sounds-in-Words and Sounds-in-Sentences, along with stimulability assessments to determine a participant's ability to produce targeted sounds when prompted. The raw score is calculated by totaling the number of correct responses for each subtest (Sounds-in-Words and Sounds-in-Sentences). The raw score is then converted to a standard score using normative data provided in the GFTA-3 manual. The lower the score, the more severe the impairment.

Arizona Articulation Proficiency Scale (Arizona-3) is an assessment that evaluates articulation skills. It measures the production of consonant sounds across different contexts and provides detailed information about sound substitutions, omissions, and distortions. The total score measures the number of correct responses and indicates the severity of any articulatory deviations.

The Speech Intelligibility Index (SII) quantifies the potential intelligibility of speech based on acoustic properties, such as frequency and intensity. It considers the impact of background noise on speech perception and helps predict how well speech will be understood in various listening conditions. The SII score ranges from 0.0 to 1.0, where a score of 0.0 indicates that no speech information is audible to the listener and a score of 1.0 signifies that all speech information is audible.

Percentage of Intelligible Words (PIW) (or percent intelligible words (PIW)) is a measure used to assess speech intelligibility by calculating the proportion of words in a speech sample that are understood by listeners. It is expressed as a percentage and provides insight into how clearly an individual communicates. Percent Intelligible Utterances (PIU) measures the proportion of entire utterances that are understood by listeners in a speech sample. This metric focuses on complete phrases or sentences rather than individual words.

Percentage of Intelligible Syllables (PIS) assesses how many syllables in a speech sample are articulated clearly and correctly understood by listeners. This measure can be particularly useful for evaluating speech sound production at a more granular level. Percentage of Consonants Correct (PCC) is a widely used metric for evaluating articulation by calculating the proportion of correctly articulated consonants in a speech sample compared to the total number produced.

Percentage of Vowels Correct (PVC) is a measure used to assess the accuracy of vowel production in speech samples. It is calculated by determining the proportion of correctly articulated vowels in relation to the total number of vowels produced. Percentage of Vowels and Diphthongs Correct (PVC-R) expands on the PVC measure by including both vowels and diphthongs in the assessment. This measure evaluates the accuracy of both types of sounds in a speech sample, providing a more comprehensive view of a speaker's vowel sound production.

### Example 3: Use of Application to Individuals with Fluency Pathologies

In one example, the application (e.g., the application 125) will be given to individuals who have various speech pathologies per International Classification of Diseases 11th Revision (ICD-11) or Diagnostic and Statistical Manual of Mental Disorders, Fifth Edition (DSM-5). The fluency pathologies may include stuttering and cluttering, among others. The speech pathologies can be of mild-to-moderate impairment. The treatment will be 5 days to 6 months long. Improvements in fluency pathologies will be shown, as measured by a Stuttering Severity Instrument (SSI-4), Overall Assessment of the Speaker's Experience of Stuttering (OASES), or a metric based on the speech classification (e.g., the classification 305), among others, over the course of using the application. Other metrics can be used to show improvements, such as those detailed herein in other Examples (e.g., Examples 2 and 4-6).

Stuttering Severity Instrument, Fourth Edition (SSI-4) is an assessment tool used to evaluate the severity of stuttering. It measures stuttering across four main areas: (1) Frequency (e.g., percentage of syllables that are stuttered, calculated from speech samples); (2) Duration (e.g., average length of the three longest stuttering events, timed to the nearest tenth of a second); (3) Physical Concomitants (e.g., observations of secondary behaviors associated with stuttering, such as facial grimaces, head movements, and distracting sounds); (4) naturalness of speech (e.g., an evaluation of how typical the speaker's speech sounds compared to peers). Each component is scored independently and contributes to an overall severity rating, which can range from very mild to severe.

Overall Assessment of the Speaker's Experience of Stuttering (OASES) is a comprehensive self-report tool designed to evaluate the impact of stuttering on an individual's life. It assesses various dimensions, such as perceptions of stuttering (how individuals view their stuttering behaviors and their emotional responses); communication in daily life (the extent to which stuttering affects social interactions, academic performance, and overall quality of life); and reactions to stuttering (how individuals cope with their stuttering in different contexts), among others.

### Example 4: Use of Application to Individuals with Voice Pathologies

In one example, the application (e.g., the application 125) will be given to individuals who have voice pathologies per International Classification of Diseases 11th Revision (ICD-11) or Diagnostic and Statistical Manual of Mental Disorders, Fifth Edition (DSM-5). The voice pathologies may include, for example, spasmodic dysphonia, vocal cord paralysis, laryngitis, and dysphonia, among others. The speech pathologies can be of mild-to-moderate impairment. The treatment will be 5 days to 6 months long. Improvements voice pathologies will be shown, as measured by maximum phonation time (MPT), GRBAS scale, vocal range profile (VRP), voice handicap index (VHI), Voice Related Quality of Life (V-RQOL), Consensus Auditory-Perceptual Evaluation of Voice (CAPE-V), Diadochokinetic Rate (DDK), prosody voice screening profile (PVSP), or a metric based on the speech classification (e.g., the classification 305), among others, over the course of using the application. Other metrics can be used to show improvements, such as those detailed herein in other (e.g., Examples 2, 3, 5, and 6).

Maximum Phonation Time (MPT) is a measure of the longest duration a person can sustain a vowel sound on one breath at a comfortable pitch and loudness. Assessed using the vowel sound (e.g., "ah"), the MPT is used to evaluate voice quality and monitor changes over time. The MPT may be performed by timing the subject's attempts, and using the longest of the three attempts as the score. For adults, a normal MPT is typically 15-25 seconds for females and 25-35 seconds for males.

GRBAS Scale is used to evaluate and rate the perceptual quality of a participant's voice. The scale evaluates five key aspects of voice, with each aspect rated on a scale from 0 to 3, where 0 is normal (no perceived deviation) and 3 is severe. The components of the GRBAS scale include: Grade (G) measuring overall severity of the voice disorder; Roughness (R) measuring irregularity in vocal fold vibration and reflecting the perception of a harsh, irregular sound in the voice, often associated with uneven vocal fold vibration; Breathiness (B) measuring audible air escape in the voice and indicating how much excess air escapes during phonation, producing a soft or airy quality, often due to incomplete vocal fold closure; and Asthenia (A) referring to weakness or lack of power in the voice and capturing the perception of vocal weakness or reduced vocal energy that can affect loudness and clarity; and Strain (S) associated with perception of vocal effort or tension and measuring the degree of strain or tension in the voice, often resulting from excessive vocal effort or hyperfunction.

Vocal Range Profile (VRP) (also known as a phonetogram) is an objective assessment of an individual's vocal capabilities, capturing the range of pitches and sound pressure levels (SPL) that can be produced. This profile is generated by mapping the fundamental frequency (F0) against intensity across the vocal range, creating a comprehensive representation of both pitch and loudness dynamics.

Voice Handicap Index (VHI) is a validated, self-assessment tool designed to quantify the psychological and functional impact of voice disorders on an individual's quality of life. It comprises 30 items across three domains: functional, physical, and emotional, which collectively gauge the perceived severity of the voice handicap. Scoring each item on a Likert scale, the VHI provides a quantitative measure for assessing the patient's subjective experience of their voice disorder.

Voice-Related Quality of Life (V-RQOL) is a validated, voice-specific patient-reported outcome measure developed to evaluate the impact of voice disorders on a patient's perceived quality of life. Including 10 items, the V-RQOL questionnaire assesses functional, emotional, and social aspects of voice-related quality of life, yielding scores that reflect the degree to which a voice disorder affects daily life and well-being.

Consensus Auditory-Perceptual Evaluation of Voice (CAPE-V) is a standardized tool for the perceptual assessment of vocal quality. It evaluates key parameters of voice, including overall severity, roughness, breathiness, strain, pitch, and loudness. Using a visual analog scale, the CAPE-V rates the severity of each parameter based on structured speech tasks.

The Diadochokinetic Rate (DDK) refers to the measurement of an individual's ability to produce rapid alternating movements of speech sounds. This assessment typically involves having subjects repeat syllables such as "pa-ta-ka" as quickly as possible within a specified time frame. The DDK rate quantifies the speed and coordination of oral motor skills essential for fluent speech production. It serves as an indicator of motor control related to speech.

Prosody-Voice Screening Profile (PVSP) is a standardized assessment tool designed to evaluate prosodic and vocal characteristics in conversational speech. The PVSP incorporates perceptual judgments across seven suprasegmental domains: phrasing, rate, stress, loudness, pitch, laryngeal quality, and resonance. Each domain is typically scored using a scale that may range from 0 to 5 or similar, where 0 indicates no issues or typical performance and 5 scores indicate increasing severity of prosodic or vocal abnormalities. The scores from each domain are summed to provide an overall score that reflects the individual's prosodic and voice characteristics. Higher total scores indicate greater difficulties in prosody and voice, while lower scores suggest more typical performance.

### Example 5: Use of Application to Individuals with Resonance Pathologies

In one example, the application (e.g., the application 125) will be given to individuals who have resonance pathologies per International Classification of Diseases 11th Revision (ICD-11) or Diagnostic and Statistical Manual of Mental Disorders, Fifth Edition (DSM-5). The resonance pathologies may include hypernasality, hyponasality, and velopharyngeal dysfunction, among others. The speech pathologies can be of mild-to-moderate impairment. The treatment will be 5 days to 6 months long. Improvements in resonance pathologies will be shown, as measured by Bzoch Hypernasality Scale, Resonance Severity Index, nasalance score, a metric based on the speech classification (e.g., the classification 305), among others, over the course of using the application. Other metrics can be used to show improvements, such as those detailed herein in other Examples (e.g., Examples 2-4 and 6).

Bzoch Hypernasality Scale is a perceptual assessment tool used to evaluate the severity of hypernasality in speech. It provides a systematic way for clinicians to rate the degree of hypernasality based on auditory-perceptual judgments. The scale typically ranges from normal resonance (0) to severe hypernasality (4), allowing for standardized documentation of speech characteristics associated with velopharyngeal dysfunction.

Resonance Severity Index is a quantitative measure used to assess the severity of resonance disorders, particularly hypernasality and hyponasality. This index combines various assessment tools, including perceptual ratings and instrumental measures such as nasometry, to provide an overall score that reflects the severity of resonance issues.

Nasalance score is a numerical value derived from nasometric measurements that quantifies the relative amount of nasal acoustic energy present in speech. It is calculated as the ratio of nasal sound energy to total sound energy (nasal plus oral), expressed as a percentage. Higher nasalance scores indicate greater nasal resonance, often associated with hypernasality, while lower scores suggest reduced nasal resonance, potentially indicating hyponasality.

### Example 6: Use of Application to Individuals with Neurological Speech Disorders

In one example, the application (e.g., the application 125) will be given to individuals who have various speech pathologies per International Classification of Diseases 11th Revision (ICD-11) or Diagnostic and Statistical Manual of Mental Disorders, Fifth Edition (DSM-5). The neurological speech pathologies may include aphasia, apraxia of speech, and dysarthria, among others. The speech pathologies can be of mild-to-moderate impairment. The treatment will be 5 days to 6 months long. Improvements in defect in the neurological speech disorder will be shown, as measured by Western Aphasia Battery (WAB), Boston Diagnostic Aphasia Examination (BDAE), Communicative Effectiveness Index (CETI), Apraxia Battery for Adults (ABA-2), DDK rate, Percentage of Consonants Correct-Revised (PCC-R), Frenchay Dysarthria Assessment (FDA-2), and Dysarthria Impact Profile (DIP), and a metric based on the speech classification (e.g., the classification 305), among others, over the course of using the application. Other metrics can be used to show improvements, such as those detailed herein in other Examples (e.g., Example 2-5).

Western Aphasia Battery (WAB) is a comprehensive, standardized assessment that evaluates language function across domains including spontaneous speech, auditory comprehension, repetition, and naming. The WAB yields an Aphasia Quotient (AQ) and Cortical Quotient (CQ). AQ is a composite score quantifying a participant's language abilities across key areas, including spontaneous speech, auditory comprehension, repetition, and naming. The score ranges from 0 to 100, where higher scores indicate better language function. CQ is a broader composite score evaluating overall cortical function. CQ is derived by combining scores from language and non-language subtests, providing a measure of overall cortical or cognitive impairment The score also ranges from 0 to 100, with higher scores indicating better cognitive function.

Boston Diagnostic Aphasia Examination (BDAE) assesses fluency, comprehension, repetition, and other language abilities, producing a detailed profile of aphasic symptoms. The BDAE enables differential diagnosis of aphasia types and tracks language recovery, supporting targeted therapeutic interventions. The BDAE is divided into several subtests, each focusing on specific language modalities. Fluency is assessed through spontaneous speech where features such as melodic line, phrase length, articulatory agility, grammatical form, paraphasia, and word-finding abilities. Auditory Comprehension is evaluated on the ability to understand spoken language through various tasks. Oral Expression is assessed for naming abilities, sentence completion, and the use of automatized sequences. Reading and Writing involves tasks such as reading words and sentences aloud, as well as writing tasks that evaluate spelling and narrative skills. The BDAE includes an Aphasia Severity Rating Scale, which provides an overall score reflecting the severity of language impairment.

Communicative Effectiveness Index (CETI) is a rating scale designed to assess the functional communication abilities of individuals with aphasia, particularly following a stroke. The CETI evaluates an individual's overall capacity to convey meaning and comprehend messages in everyday situations using any available communication methods. It comprises a series of items that reflect common communicative scenarios, allowing caregivers or significant others to rate the individual's performance in these contexts. CETI is rated on a Likert scale, typically from 1 (cannot perform) to 10 (performs very well), based on the observed or reported effectiveness of communication in each scenario

Apraxia Battery for Adults (ABA-2) is an assessment tool designed to evaluate the presence and severity of apraxia of speech in adolescents and adults. This battery comprises six subtests that assess various aspects of speech production, including articulatory precision, phonemic sequencing, and the ability to produce both automatic and volitional speech tasks.

Percentage of Consonants Correct-Revised (PCC-R) quantifies speech sound accuracy by measuring correct consonant productions in a speech sample. Lower PCC-R scores in apraxia of speech reflect impaired motor planning, contributing to overall articulatory inaccuracy.

Frenchay Dysarthria Assessment (FDA-2) is an evaluation tool that measures speech production across domains such as reflexes, respiration, phonation, and articulation. FDA-2 scores categorize the type and severity of dysarthria, assisting in differential diagnosis and therapeutic planning.

Dysarthria Impact Profile (DIP) is a self-report measure evaluating the physical, emotional, and social impact of dysarthria on the individual's quality of life. The DIP comprises multiple items that are categorized into specific domains, including self-perception, social interactions, and the overall impact of dysarthria on daily life. It aims to quantify how dysarthria affects an individual's self-esteem, self-concept, and interpersonal relationships.

Improvements to the diagnostic values provided above account for user speech expressiveness abilities, such that improvements in user speech expressiveness will result in improved diagnostic values. Thus, an improvement in any one of the diagnostic values following treatment with the protocol described herein indicates an improvement in the user's speech expressiveness.

### Example 7: Use of Application to Individuals with Autism Spectrum Disorder (ASD)

In one example, the application (e.g., the application 125) will be given to individuals who have autism spectrum disorder (ASD) per International Classification of Diseases 11th Revision (ICD-11) or Diagnostic and Statistical Manual of Mental Disorders, Fifth Edition (DSM-5). The ASD 7 may be mild to moderate severity. The treatment will be 5 days to 6 months long. Improvements in defect in speech expressiveness will be shown, as measured by Autism Diagnostic Observation Schedule (ADOS), Test of Pragmatic Language (TOPL-2), CETI, Social Responsiveness Scale (SRS-2), Comprehensive Assessment of Spoken Language (CASL-2), Functional Communication Profile (FCP-R), and a metric based on the speech classification (e.g., the classification 305), among others, over the course of using the application. Other metrics can be used to show improvements, such as those detailed herein in other Examples.

Autism Diagnostic Observation Schedule (ADOS) is a standardized assessment tool used to diagnose ASDs. The ADOS comprises a series of structured and semi-structured tasks that facilitate direct observation of social interaction, communication, and play behaviors. It includes five modules tailored to the individual's developmental and language levels, allowing examiners to assess behaviors relevant to autism diagnosis. Behaviors observed during the assessment are scored on a 0-3 scale, with 0 indicating typical development and 3 indicating severe impairment. These scores are totaled across domains to yield a composite score that is compared to diagnostic cutoffs for ASD. Higher composite scores indicate greater severity of ASD symptoms.

Test of Pragmatic Language (TOPL-2) is a standardized tool that assesses pragmatic language skills. It evaluates abilities such as understanding conversational norms, recognizing context, and adjusting language based on social interactions, critical skills for social communication. Responses are scored based on accuracy and appropriateness, with each item scored on a scale. The scores are compiled to create a Total Pragmatic Language score, which is then compared to normative data. Percentile ranks and standard scores help interpret the individual's performance relative to age-matched peers, with lower scores indicating more significant difficulties in pragmatic language use.

Communicative Effectiveness Index (CETI) is a rating scale designed to assess the functional communication abilities of individuals with aphasia, particularly following a stroke. The CETI evaluates an individual's overall capacity to convey meaning and comprehend messages in everyday situations using any available communication methods. It comprises a series of items that reflect common communicative scenarios, allowing caregivers or significant others to rate the individual's performance in these contexts. CETI is rated on a Likert scale, typically from 1 (low performance) to 10 (high performance).

Social Responsiveness Scale (SRS-2) is a rating scale that evaluates social communication, social awareness, and repetitive behaviors associated with autism spectrum disorder. Each item is rated on a 4-point Likert scale from 1 (not true) to 4 (almost always true), capturing the frequency of observed social and communicative behaviors. Scores are totaled to produce a T-score that reflects the severity of social impairment, with higher T-scores indicating more significant social challenge.

Comprehensive Assessment of Spoken Language (CASL-2) is a standardized test that evaluates language skills across areas such as comprehension, expression, syntax, semantic knowledge, and pragmatic language. CASL-2 assesses an individual's spoken language abilities in a structured format to aid in the diagnosis and intervention planning for language disorders. Each subtest score is based on the number of correct responses, which are converted to standard scores. Subtest scores can be combined to yield composite scores for broader language domains (e.g., syntax, semantics). These composite scores are interpreted using normative data, with lower scores indicating greater language impairment.

Functional Communication Profile (FCP-R) is an assessment tool that evaluates an individual's functional communication abilities across various contexts. It focuses on how effectively a person communicates in daily life situations rather than solely on formal language skills. The FCP-R assesses receptive and expressive communication skills through observations and caregiver reports, providing insights into the individual's strengths and challenges in functional communication settings. Each communication domain in the FCP-R is rated based on observed or reported communication behaviors, typically using a rating scale from 1 (cannot perform) to 5 (performs independently). Scores are compiled across domains to create a profile of communication skills, with higher scores indicating greater functional independence in communication.

Improvements to the diagnostic values provided above account for user speech expressiveness abilities, such that improvements in user speech expressiveness will result in improved diagnostic values. Thus, an improvement in any one of the diagnostic values following treatment with the protocol described herein indicates an improvement in the user's speech expressiveness.

### Example 8: Use of Application to Individuals with Multiple Sclerosis

In one example, the Application will be given to individuals who have multiple sclerosis (MS) per International Classification of Diseases 11th Revision (ICD-11) or Diagnostic and Statistical Manual of Mental Disorders, Fifth Edition (DSM-5). The treatment will be 5 days to 6 months long. Improvements in defect in speech expressiveness will be shown, as measured by a metric based on the speech classification (e.g., the classification 305), among others, over the course of using the application. Other metrics can be used to show improvements, such as those detailed herein in other Examples (e.g., Example 6). Improvements to the diagnostic values provided herein account for user speech expressiveness abilities, such that improvements in user speech expressiveness will result in improved diagnostic values. Thus, an improvement in any one of the diagnostic values following treatment with the protocol described herein indicates an improvement in the user's speech expressiveness.

### Example 9: Use of Application to Individuals with Affective Disorders

In one example, the application (e.g., the application 125) will be given to individuals who have affective disorders per International Classification of Diseases 11th Revision (ICD-11) or Diagnostic and Statistical Manual of Mental Disorders, Fifth Edition (DSM-5). The treatment will be 5 days to 6 months long. Improvements in defect in speech expressiveness will be shown, as measured by a Hamilton Depression Rating Scale (Ham-D) or a metric based on the speech classification (e.g., the classification 305), among others, over the course of using the application. Other metrics can be used to show improvements, such as those detailed herein in other Examples.

Hamilton Rating Scale for Depression (HAM-D) is a clinician-administered scale that assesses the severity of depressive symptoms, including changes in speech expressiveness associated with depression. The HAM-D evaluates a range of depressive features across domains such as mood, psychomotor activity, and cognitive function, with specific items assessing speech-related symptoms like psychomotor retardation and diminished verbal output. These speech characteristics (e.g., often presenting as slowed speech rate, reduced vocal inflection, and decreased spontaneous verbal engagement) are scored on a Likert scale from 0 (absent) to 4 (severe), contributing to an overall depression severity score.

Improvements to the diagnostic values provided above account for user speech expressiveness abilities, such that improvements in user speech expressiveness will result in improved diagnostic values. Thus, an improvement in any one of the diagnostic values following treatment with the protocol described herein indicates an improvement in the user's speech expressiveness.

### Example 10: Use of Application to Individuals with Neurodegenerative Diseases

In one example, the application (e.g., the application 125) will be given to individuals who have a neurodegenerative disease, such as dementia, Alzheimer's disease, or Parkinson's disease per International Classification of Diseases 11th Revision (ICD-11) or Diagnostic and Statistical Manual of Mental Disorders, Fifth Edition (DSM-5). The treatment will be 5 days to 6 months long. Improvements in defect in speech expressiveness will be shown, as measured by a metric based on the speech classification (e.g., the classification 305), among others, over the course of using the application. Other metrics can be used to show improvements, such as those detailed herein in other Examples (e.g., Example 6). Improvements to the diagnostic values provided herein account for user speech expressiveness abilities, such that improvements in user speech expressiveness will result in improved diagnostic values. Thus, an improvement in any one of the diagnostic values following treatment with the protocol described herein indicates an improvement in the user's speech expressiveness.

### Example 11: Use of Application to Individuals with Schizophrenia

In one example, the application (e.g., the application 125) will be given to individuals who have schizophrenia per International Classification of Diseases 11th Revision (ICD-11) or Diagnostic and Statistical Manual of Mental Disorders, Fifth Edition (DSM-5), are experiencing mild-to-moderate functional impairment as evidenced by WHO-DAS 2.0, and are prescribed antipsychotic medication(s). The treatment will be 5 days to 6 months long. Improvements in defect in speech expressiveness will be shown, as measured by a MAP-SR value, SEACS metric social effort value, SEACS metric for social consciousness value, or a metric based on the speech classification (e.g., the classification 305), among others, over the course of using the application. Other metrics can be used to show improvements, such as those detailed herein in other Examples.

The Motivation and Pleasure Scale-Self Report (MAP-SR) is a self-report tool derived from the Clinical Assessment Interview for Negative Symptoms that assesses the motivation and pleasure domain of negative symptoms in patients with psychotic disorders. The scale includes 15 items that record motivation, effort, interest and pleasure in different areas of life. All items are rated on a 5-point Likert scale, where lower scores reflect greater severity.

The Social Effort and Conscientiousness Scale (SEACS) is a self-report measure of effortful behavior in the service of forming and maintaining social bonds. The questionnaire includes 17 items that are rated on a 6-point Likert scale. The SEACS is broken into 2 subscales. The Social Effort subscale reflects tendencies towards effort exertion in the service of social connection for one's own purpose, while the Social Conscientiousness subscale reflects tendencies towards effort exertion in the service of adhering to social norms.

Improvements to the diagnostic values provided above account for user speech expressiveness abilities, such that improvements in user speech expressiveness will result in improved diagnostic values. Thus, an improvement in any one of the diagnostic values following treatment with the protocol described herein indicates an improvement in the user's speech expressiveness.

### C. Network and Computing Environment

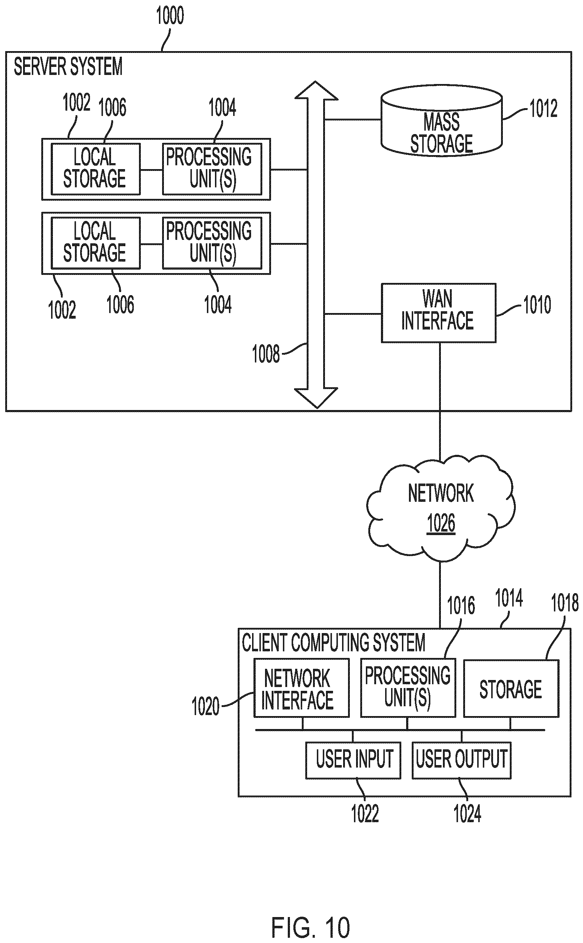

Various operations described herein can be implemented on computer systems. FIG. 10 shows a simplified block diagram of a representative server system1000, client computer system1014, and network1026 usable to implement certain embodiments of the present disclosure. In various embodiments, server system1000 or similar systems can implement services or servers described herein or portions thereof. Client computer system1014 or similar systems can implement clients described herein. The system 100 described herein can be similar to the server system1000. Server system1000 can have a modular design that incorporates a number of modules1002 (e.g., blades in a blade server embodiment); while two modules1002 are shown, any number can be provided. Each module1002 can include processing unit(s)1004 and local storage 1 006.

Processing unit(s)1004 can include a single processor, which can have one or more cores, or multiple processors. In some embodiments, processing unit(s)1004 can include a general-purpose primary processor as well as one or more special-purpose co-processors such as graphics processors, digital signal processors, or the like. In some embodiments, some or all processing units1004 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s)1004 can execute instructions stored in local storage1006. Any type of processors in any combination can be included in processing unit(s)1004.

Local storage1006 can include volatile storage media (e.g., DRAM, SRAM, SDRAM, or the like) and/or non-volatile storage media (e.g., magnetic or optical disk, flash memory, or the like). Storage media incorporated in local storage1006 can be fixed, removable, or upgradeable as desired. Local storage 1 006 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s)1004 need at runtime. The ROM can store static data and instructions that are needed by processing unit(s)1004. The permanent storage device can be a non-volatile read-and-write memory device that can store instructions and data even when module1002 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage1006 can store one or more software programs to be executed by processing unit(s)1004, such as an operating system and/or programs implementing various server functions such as functions of the system 100 or any other system described herein, or any other server(s) associated with system 100 or any other system described herein.

"Software" refers generally to sequences of instructions that, when executed by processing unit(s)1004, cause server system1000 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s)1004. Software can be implemented as a single program or a collection of separate programs or program modules that interact as desired. From local storage 1 006 (or non-local storage described below), processing unit(s)1004 can retrieve program instructions to execute and data to process in order to execute various operations described above.

In some server systems 1000, multiple modules 1002 can be interconnected via a bus or other interconnect1008, forming a local area network that supports communication between modules1002 and other components of server system1000. Interconnect1008 can be implemented using various technologies, including server racks, hubs, routers, etc.

A wide area network (WAN) interface1010 can provide data communication capability between the local area network (e.g., through the interconnect1008) and the network1026, such as the Internet. Other technologies can be used to communicatively couple the server system with the network1026, including wired (e.g., Ethernet, IEEE 802.3 standards) and/or wireless technologies (e.g., Wi-Fi, IEEE 802.11 standards).

In some embodiments, local storage1006 is intended to provide working memory for processing unit(s)1004, providing fast access to programs and/or data to be processed while reducing traffic on interconnect1008. Storage for larger quantities of data can be provided on the local area network by one or more mass storage subsystems1012 that can be connected to interconnect1008. Mass storage subsystem1012 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem1012. In some embodiments, additional data storage resources may be accessible via WAN interface1010 (potentially with increased latency).

Server system 1 000 can operate in response to requests received via WAN interface 1 010. For example, one of modules1002 can implement a supervisory function and assign discrete tasks to other modules 1002 in response to received requests. Work allocation techniques can be used. As requests are processed, results can be returned to the requester via WAN interface1010. Such operation can generally be automated. Further, in some embodiments, WAN interface1010 can connect multiple server systems1000 to each other, providing scalable systems capable of managing high volumes of activity. Other techniques for managing server systems and server farms (collections of server systems that cooperate) can be used, including dynamic resource allocation and reallocation.

Server system 1 000 can interact with various user-owned or user-operated devices via a wide-area network such as the Internet. An example of a user-operated device is shown in FIG. 10 as client computing system1014. Client computing system1014 can be implemented, for example, as a consumer device such as a smartphone, other mobile phone, tablet computer, wearable computing device (e.g., smart watch, eyeglasses), desktop computer, laptop computer, and so on.

For example, client computing system1014 can communicate via WAN interface 1 010. Client computing system1014 can include computer components such as processing unit(s)1016, storage device1018, network interface1020, user input device 1 022, and user output device1024. Client computing system1014 can be a computing device implemented in a variety of form factors, such as a desktop computer, laptop computer, tablet computer, smartphone, other mobile computing device, wearable computing device, or the like.

Processing unit1016 and storage device1018 can be similar to processing unit(s)1004 and local storage1006 described above. Suitable devices can be selected based on the demands to be placed on client computing system1014. For example, client computing system1014 can be implemented as a "thin" client with limited processing capability or as a high-powered computing device. Client computing system 10 14 can be provisioned with program code executable by processing unit(s)1016 to enable various interactions with server system 1000.

Network interface1020 can provide a connection to the network1026, such as a wide area network (e.g., the Internet) to which WAN interface1010 of server system1000 is also connected. In various embodiments, network interface1020 can include a wired interface (e.g., Ethernet) and/or a wireless interface implementing various RF data communication standards such as Wi-Fi, Bluetooth, or cellular data network standards (e.g., 3G, 4G, LTE, etc.).

User input device1022 can include any device (or devices) via which a user can provide signals to client computing system1014; client computing system1014 can interpret the signals as indicative of particular user requests or information. In various embodiments, user input device 1 022 can include any or all of a keyboard, touch pad, touch screen, mouse or other pointing device, scroll wheel, click wheel, dial, button, switch, keypad, microphone, and so on.

User output device 1 024 can include any device via which client computing system1014 can provide information to a user. For example, user output device1024 can include display-to-display images generated by or delivered to client computing system1014. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices1024 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s)1004 and1016 can provide various functionality for server system1000 and client computing system1014, including any of the functionality described herein as being performed by a server or client, or other functionality.

It will be appreciated that server system1000 and client computing system1014 are illustrative and that variations and modifications are possible. Computer systems used in connection with embodiments of the present disclosure can have other capabilities not specifically described here. Further, while server system1000 and client computing system1014 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

Aspects of the present disclosure are set out in the following numbered clauses, in which:
1. A method of providing instructions for speech based on speech classifications of verbal communications from users, comprising:
   identifying, by one or more processors, a first audio sample of a first verbal communication from a user;
   generating, by the one or more processors, a first plurality of speech characteristics for the first verbal communication using the first audio sample;
   determining, by the one or more processors, from a plurality of speech classifications, a first speech classification for the first verbal communication based on the first plurality of speech characteristics;
   selecting, by the one or more processors, from a plurality of actions, an action comprising modifying one or more of the speech characteristics to define an utterance for the user based on the first speech classification; and
   providing, by the one or more processors, an instruction presenting a message to prompt the user to perform the utterance defined by the action selected from the plurality of actions.
2. The method of clause 1, wherein determining the first speech classification further comprises determining the verbal communication is not able to be understood, and
   wherein selecting the action further comprises selecting the action for the user to modify at least one of the first plurality of speech characteristics in the utterance.
3. The method of clause 1, wherein determining the first speech classification further comprises determining the verbal communication is able to be understood, and
   wherein selecting the action further comprises selecting the action for the user to maintain one or more of the first plurality of speech characteristics in the utterance.
4. The method of clause 1, wherein the determining the first plurality of speech characteristics further comprises generating a score indicating a degree of severity of at least one of the first plurality of the speech characteristics, and
   wherein providing the instruction further comprises providing the instruction including the message to identify the score for presentation to the user.
5. The method of clause 1, wherein the first plurality of speech characteristics further comprises a corresponding plurality of scores, each of the plurality of scores defined along a scale for a respective speech characteristic.
6. The method of clause 5, wherein the determining the first speech classification further comprises determining the first speech classification based on at least one of: (i) an average of the plurality of scores, (ii) a weighted combination of the plurality of scores, (iii) a comparison with a dataset comprised of a second plurality of scores, (iv) a neural network model, or (v) a generative transformer model.
7. The method of clause 1, wherein the determining the first speech classification further comprises applying a machine learning (ML) model to the first plurality of speech characteristics, wherein the ML model is established using a training dataset comprising a plurality of examples, each of the plurality of examples identifying (i) a respective second audio sample of a second verbal communication and (ii) a respective second classification from the plurality of speech classifications.
8. The method of clause 1, wherein providing the instruction further comprises providing the instruction including the message to identify at least one of (i) one or more of the first plurality of speech characteristics and (ii) the action to modify the utterance.
9. The method of clause 1, further comprising identifying, by the one or more processors, from a plurality of factors, a factor as causing the first speech classification based on at least one of the first plurality of speech characteristics; and
   wherein providing the instruction further comprises providing the message to identify the factor as the cause of the first speech characteristic.
10. The method of clause 1, further comprising generating, by the one or more processors, for playback to the user, a second audio sample by modifying the first audio sample in accordance with the action.
11. The method of clause 10, wherein generating the second audio sample further comprises applying a speech synthesis model to the first audio sample and the action to generate the second audio sample.
12. The method of clause 1, further comprising:
   determining, by the one or more processors, a second speech classification for a second verbal communication of the user based on a second plurality of speech characteristics, the second plurality of speech characteristics generated from a second audio sample identified at a time subsequent to provision of the instruction; and
   determining, by the one or more processors, a progress metric based on a comparison between the first speech classification from prior to the instruction and the second speech classification subsequent to the provision of the instruction.
13. The method of clause 1, wherein the first plurality of speech characteristics further comprises at least one of: (i) respiration, (ii) phonation, (iii) articulation, (iv) resonance, (v) prosody, (vii) pitch, (viii) jitter, (ix) shimmer, (x) rhythm, (xi) pacing, or (xii) pausing.
14. The method of clause 1, wherein the plurality of speech classifications comprises at least one of: (i) mumbling, (ii) lisping, (iii) dysarthria, (vi) stuttering, or (v) understandable.
15. The method of clause 1, further comprising:
   identifying, by the one or more processors, a first video sample of a first non-verbal communication from the user, at least in partial concurrence with the first verbal communication;
   determining, by the one or more processors, a first plurality of non-verbal characteristics of the first non-verbal communication using the first video sample, the first plurality of non-verbal characteristics including at least one of a gesture, a facial expression, an eye contact by the user; and
   wherein determining the first speech classification further comprises determining the first speech classification based on the first plurality of non-verbal characteristics.
16. The method of clause 1, wherein the user is affected by at least one of a speech impairment or a language impairment, and is undergoing speech therapy at least partially concurrently with the provision of the instruction.
17. The method of clause 1, wherein the user is affected by a disorder associated with a speech impairment and is on a medication for the disorder at least partially concurrently with the provision of the instruction.
18. A system for providing instructions for speech based on speech classifications of verbal communications from users, comprising:
   one or more processors coupled with memory, configured to:
   identify a first audio sample of a first verbal communication from a user;
   generate a first plurality of speech characteristics for the first verbal communication using the first audio sample;
   determine, from a plurality of speech classifications, a first speech classification for the first verbal communication based on the first plurality of speech characteristics;
   select, from a plurality of actions, an action comprising modifying one or more of the speech characteristics to define an utterance for the user based on the first speech classification; and
   provide an instruction presenting a message to prompt the user to perform the utterance defined by the action selected from the plurality of actions.
19. The system of clause 18, wherein the one or more processors are further configured to:
   determine the verbal communication is not able to be understood, and
   select the action for the user to modify at least one of the first plurality of speech characteristics in the utterance.
20. The system of clause 18, wherein the one or more processors are further configured to:
   determine the verbal communication is able to be understood, and
   select the action for the user to maintain one or more of the first plurality of speech characteristics in the utterance.
21. The system of clause 18, wherein the one or more processors are further configured to:
   generate a score indicating a degree of severity of at least one of the first plurality of the speech characteristics, and
   provide the instruction including the message to identify the score for presentation to the user.
22. The system of clause 18, wherein the first plurality of speech characteristics further comprises a corresponding plurality of scores, each of the plurality of scores defined along a scale for a respective speech characteristic.
23. The system of clause 22, wherein the one or more processors are further configured to determine the first speech classification based on at least one of: (i) an average of the plurality of scores, (ii) a weighted combination of the plurality of scores, (iii) a comparison with a dataset comprised of a second plurality of scores, (iv) a neural network model, or (v) a generative transformer model.
24. The system of clause 18, wherein the one or more processors are further configured to apply a machine learning (ML) model to the first plurality of speech characteristics, wherein the ML model is established using a training dataset comprising a plurality of examples, each of the plurality of examples identifying (i) a respective second audio sample of a second verbal communication and (ii) a respective second classification from the plurality of speech classifications.
25. The system of clause 18, wherein the one or more processors are further configured to provide the instruction including the message to identify at least one of (i) one or more of the first plurality of speech characteristics and (ii) the action to modify the utterance.
26. The system of clause 18, wherein the one or more processors are further configured to:
   identify, from a plurality of factors, a factor as causing the first speech classification based on at least one of the first plurality of speech characteristics; and
   provide the instruction including the message to identify the factor as the cause of the first speech characteristic.
27. The system of clause 18, wherein the one or more processors are further configured to generate, for playback to the user, a second audio sample by modifying the first audio sample in accordance with the action.
28. The system of clause 18, wherein the one or more processors are further configured to apply a speech synthesis model to the first audio sample and the action to generate the second audio sample.
29. The system of clause 18, wherein the one or more processors are further configured to:
   determine a second speech classification for a second verbal communication of the user based on a second plurality of speech characteristics, the second plurality of speech characteristics generated from a second audio sample identified at a time subsequent to provision of the instruction; and
   determine a progress metric based on a comparison between the first speech classification from prior to the instruction and the second speech classification subsequent to the provision of the instruction.
30. The system of clause 18, wherein the first plurality of speech characteristics further comprises at least one of: (i) respiration, (ii) phonation, (iii) articulation, (iv) resonance, (v) prosody, (vii) pitch, (viii) jitter, (ix) shimmer, or (x) rhythm.
31. The system of clause 18, wherein the plurality of speech classifications comprises at least one of: (i) mumbling, (ii) lisping, (iii) dysarthria, (vi) stuttering, or (v) understandable.
32. The system of clause 18, wherein the one or more processors are further configured to:
   identify a first video sample of a first non-verbal communication from the user, at least in partial concurrence with the first verbal communication;
   determine a first plurality of non-verbal characteristics of the first non-verbal communication using the first video sample, the first plurality of non-verbal characteristics including at least one of a gesture or an eye contact by the user; and
   determine the first speech classification based on the first plurality of non-verbal characteristics.
33. The system of clause 18, wherein the user is affected by at least one of a speech impairment or a language impairment, and is undergoing speech therapy at least partially concurrently with the provision of the instruction.
34. The system of clause 18, wherein the user is affected by a disorder associated with a speech impairment and is on a medication for the disorder at least partially concurrently with the provision of the instruction.
35. A method of providing instructions for speech based on characteristics of verbal communications from users, comprising:
   identifying, by one or more processors, a first audio sample of a first verbal communication from a user;
   generating, by the one or more processors, a first plurality of speech characteristics for the first verbal communication using the first audio sample;
   selecting, by the one or more processors, from a plurality of actions, an action to modify one or more of the first plurality of speech characteristics to define an utterance for the user; and
   providing, by the one or more processors, an instruction presenting a message to prompt the user to perform the utterance defined by the action selected from the plurality of actions.
36. The method of clause 35, wherein determining the first speech classification further comprises determining the verbal communication is not able to be understood, and
   wherein selecting the action further comprises selecting the action for the user to modify at least one of the first plurality of speech characteristics in the utterance.
37. The method of clause 35, wherein determining the first speech classification further comprises determining the verbal communication is able to be understood, and
   wherein selecting the action further comprises selecting the action for the user to maintain one or more of the first plurality of speech characteristics in the utterance.
38. The method of clause 35, wherein the determining the first plurality of speech characteristics further comprises generating a score indicating a degree of severity of at least one of the first plurality of the speech characteristics, and
   wherein providing the instruction further comprises providing the instruction including the message to identify the score for presentation to the user.
39. The method of clause 35, wherein the first plurality of speech characteristics further comprises a corresponding plurality of scores, each of the plurality of scores defined along a scale for a respective speech characteristic.
40. The method of clause 35, wherein the determining the first action further comprises determining the first speech classification based on at least one of: (i) an average of the plurality of scores, (ii) a weighted combination of the plurality of scores, (iii) a comparison with a dataset comprised of a second plurality of scores, (iv) a neural network model, or (v) a generative transformer model.
41. The method of clause 35, wherein the determining the first action further comprises applying a machine learning (ML) model to the first plurality of speech characteristics, wherein the ML model is established using a training dataset comprising a plurality of examples, each of the plurality of examples identifying (i) a respective second audio sample of a second verbal communication and (ii) a respective second classification from the plurality of speech classifications.
42. The method of clause 35, wherein providing the instruction further comprises providing the instruction including the message to identify at least one of (i) one or more of the first plurality of speech characteristics and (ii) the action to modify the utterance.
43. The method of clause 42, further comprising identifying, by the one or more processors, from a plurality of factors, a factor based on at least one of the first plurality of speech characteristics; and
   wherein providing the instruction further comprises providing the message to identify the factor.
44. The method of clause 35, further comprising generating, by the one or more processors, for playback to the user, a second audio sample by modifying the first audio sample in accordance with the action.
45. The method of clause 35, wherein generating the second audio sample further comprises applying a speech synthesis model to the first audio sample and the action to generate the second audio sample.
46. The method of clause 35, further comprising:
   determining, by the one or more processors, a second speech classification for a second verbal communication of the user based on a second plurality of speech characteristics, the second plurality of speech characteristics generated from a second audio sample identified at a time subsequent to provision of the instruction; and
   determining, by the one or more processors, a progress metric based on a comparison between the first speech classification from prior to the instruction and the second speech classification subsequent to the provision of the instruction.
47. The method of clause 35, wherein the first plurality of speech characteristics further comprises at least one of: (i) respiration, (ii) phonation, (iii) articulation, (iv) resonance, (v) prosody, (vii) pitch, (viii) jitter, (ix) shimmer, (x) rhythm, (xi) pacing, or (xii) pausing.
48. The method of clause 35, further comprising:
   identifying, by the one or more processors, a first video sample of a first non-verbal communication from the user, at least in partial concurrence with the first verbal communication;
   determining, by the one or more processors, a first plurality of non-verbal characteristics of the first non-verbal communication using the first video sample, the first plurality of non-verbal characteristics including at least one of a gesture, a facial expression, an eye contact by the user; and
   wherein determining the action further comprises determining the action based on the first plurality of non-verbal characteristics.
49. The method of clause 35, wherein the user is affected by at least one of a speech impairment or a language impairment, and is undergoing speech therapy at least partially concurrently with the provision of the instruction.
50. The method of clause 35, wherein the user is affected by a disorder associated with a speech impairment and is on a medication for the disorder at least partially concurrently with the provision of the instruction.
51. A system for providing instructions for speech based on characteristics of verbal communications from users, comprising:
   one or more processors coupled with memory, configured to:
   identify a first audio sample of a first verbal communication from a user;
   generate a first plurality of speech characteristics for the first verbal communication using the first audio sample;
   select, from a plurality of actions, an action to modify one or more of the first plurality of speech characteristics to define an utterance for the user; and
   provide an instruction presenting a message to prompt the user to perform the utterance defined by the action selected from the plurality of actions.
52. The system of clause 51, wherein the one or more processors are further configured to:
   determine the verbal communication is not able to be understood, and
   select the action for the user to modify at least one of the first plurality of speech characteristics in the utterance.
53. The system of clause 51, wherein the one or more processors are further configured to:
   determine the verbal communication is able to be understood, and
   select the action for the user to maintain one or more of the first plurality of speech characteristics in the utterance.
54. The system of clause 51, wherein the one or more processors are further configured to:
   generate a score indicating a degree of severity of at least one of the first plurality of the speech characteristics, and
   provide the instruction including the message to identify the score for presentation to the user.
55. The system of clause 51, wherein the first plurality of speech characteristics further comprises a corresponding plurality of scores, each of the plurality of scores defined along a scale for a respective speech characteristic.
56. The system of clause 51, wherein the one or more processor are further configured to determine the first speech classification based on at least one of: (i) an average of the plurality of scores, (ii) a weighted combination of the plurality of scores, (iii) a comparison with a dataset comprised of a second plurality of scores, (iv) a neural network model, or (v) a generative transformer model.
57. The system of clause 51, wherein the one or more processor are further configured to apply a machine learning (ML) model to the first plurality of speech characteristics, wherein the ML model is established using a training dataset comprising a plurality of examples, each of the plurality of examples identifying (i) a respective second audio sample of a second verbal communication and (ii) a respective second classification from the plurality of speech classifications.
58. The system of clause 51, wherein the one or more processors are further configured to provide the instruction including the message to identify at least one of (i) one or more of the first plurality of speech characteristics and (ii) the action to modify the utterance.
59. The system of clause 51, wherein the one or more processors are further configured to identify, from a plurality of factors, a factor based on at least one of the first plurality of speech characteristics; and provide the message to identify the factor.
60. The system of clause 51, wherein the one or more processors are further configured to generate, for playback to the user, a second audio sample by modifying the first audio sample in accordance with the action.
61. The system of clause 60, wherein the one or more processors are further configured to apply a speech synthesis model to the first audio sample and the action to generate the second audio sample.
62. The system of clause 51, wherein the one or more processors are further configured to:
   determine a second speech classification for a second verbal communication of the user based on a second plurality of speech characteristics, the second plurality of speech characteristics generated from a second audio sample identified at a time subsequent to provision of the instruction; and
   determine a progress metric based on a comparison between the first speech classification from prior to the instruction and the second speech classification subsequent to the provision of the instruction.
63. The system of clause 51, wherein the first plurality of speech characteristics further comprises at least one of: (i) respiration, (ii) phonation, (iii) articulation, (iv) resonance, (v) prosody, (vii) pitch, (viii) jitter, (ix) shimmer, (x) rhythm, (xi) pacing, or (xii) pausing.
64. The system of clause 51, wherein the one or more processors are further configured to:
   identify a first video sample of a first non-verbal communication from the user, at least in partial concurrence with the first verbal communication;
   determine a first plurality of non-verbal characteristics of the first non-verbal communication using the first video sample, the first plurality of non-verbal characteristics including at least one of a gesture, a facial expression, an eye contact by the user; and
   determine the action based on the first plurality of non-verbal characteristics.
65. The system of clause 51, wherein the user is affected by at least one of a speech impairment or a language impairment, and is undergoing speech therapy at least partially concurrently with the provision of the instruction.
66. The system of clause 51, wherein the user is affected by a disorder associated with a speech impairment and is on a medication for the disorder at least partially concurrently with the provision of the instruction.
67. A method of ameliorating defect of speech expressiveness in a user in need thereof, comprising:
   obtaining, by one or more processors, a first metric associated with the user prior to completion of at least one of a plurality of sessions;
   repeating, by the one or more processors, provision of the plurality of sessions to the user, each session of the plurality of sessions comprising:
      identifying a first audio sample of a first verbal communication from a user;
      generating a first plurality of speech characteristics for the first verbal communication using the first audio sample;
      determining, from a plurality of actions, an action to modify one or more of the first plurality of speech characteristics to define an utterance for the user; and
      providing an instruction presenting a message to prompt the user to perform the utterance defined by the action selected from the plurality of actions;
   obtaining, by the one or more processors, a second metric associated with the user subsequent to the completion of at least one of the plurality of sessions; and
   wherein amelioration in the defect of speech expressiveness occurs in the user, when the second metric is (i) decreased from the first metric by a first predetermined margin or (ii) increased from the first metric by a second predetermined margin.
68. The method of clause 67, wherein the user is diagnosed with a condition comprising at least one of a speech pathology, autism spectrum disorder (ASD), multiple sclerosis, a neurodegenerative disease, dementia, Parkinson's disease, Alzheimer's disease, an affective disorder, or schizophrenia.
69. The method of clause 68, wherein the user is receiving a treatment, at least in partial concurrence with the at least one of the plurality of sessions, wherein the treatment comprises at least one of a psychosocial intervention or a medication to address the condition.
70. The method of clause 68, wherein the defect of the speech expressiveness is caused by the condition.
71. The method of clause 67, wherein the user is an adult aged at least 18 years or older.
72. The method of clause 67, wherein the plurality of sessions are provided over a period of time ranging between 3 days to 6 months.
73. The method of clause 67, wherein the first verbal communication of the first audio sample comprises an utterance of one or more words by the user,
   wherein the first plurality of speech characteristics further comprises at least one of: (i) respiration, (ii) phonation, (iii) articulation, (iv) resonance, (v) prosody, (vii) pitch, (viii) jitter, (ix) shimmer, (x) rhythm, (xi) pacing, or (xii) pausing.
74. The method of clause 67, wherein at least one of the plurality of sessions further comprise:
   determining, from a plurality of speech classifications, a first speech classification for the first verbal communication based on the first plurality of speech characteristics; and
   selecting, from a plurality of actions, an action comprising modifying one or more of the speech characteristics to define an utterance for the user based on the first speech classification,
   wherein the plurality of speech classifications comprises at least one of: (i) mumbling, (ii) lisp, (iii) dysarthria, (vi) stuttering, or (v) understandable.
75. The method of clause 67, wherein the amelioration in the defect in the speech expressiveness in the user with a speech pathology occurs, when the second metric is decreased from the first metric by the first predetermined margin or when the second metric is increased from the first metric by the second predetermined margin, and wherein the first metric and the second metric are at least one of: Goldman-Fristoe Test of Articulation (GFTA-3) values, Arizona Articulation Proficiency Scale (Arizona-3) values, speech intelligibility index (SII) values, Percentage of Intelligible Words (PIW) values, Percent Intelligible Utterances (PIU) values, Percentage of Intelligible Syllables (PIS) values, Percentage of Consonants Correct (PCC) values, Percentage of Vowels Correct (PVC) values, Percentage of Vowels and Diphthongs Correct (PVC-R) values, Stuttering Severity Instrument (SSI-4) values, Overall Assessment of the Speaker's Experience of Stuttering (OASES) values, maximum phonation time (MPT) values, GRBAS scale values, vocal range profile (VRP) values, voice handicap index (VHI) values, Voice Related Quality of Life (V-RQOL) values, Consensus Auditory-Perceptual Evaluation of Voice (CAPE-V) values, Diadochokinetic Rate (DDK) values, prosody voice screening profile (PVSP) values, Bzoch Hypernasality Scale values, Resonance Severity Index values, nasalance score values, Western Aphasia Battery (WAB) values, Boston Diagnostic Aphasia Examination (BDAE) values, Communicative Effectiveness Index (CETI) values, Apraxia Battery for Adults (ABA-2) values, DDK rate, Percentage of Consonants Correct-Revised (PCC-R) values, Frenchay Dysarthria Assessment (FDA-2) values, or Dysarthria Impact Profile (DIP) values.
76. The method of clause 67, wherein the amelioration in the expressiveness of speech in the user with ASD occurs, when the second metric is decreased from the first metric by the first predetermined margin or when the second metric is increased from the first metric by the second predetermined margin, and wherein the first metric and the second metric are at least one of Autism Diagnostic Observation Schedule (ADOS) values, Test of Pragmatic Language (TOPL-2) values, CETI values, Social Responsiveness Scale (SRS-2) values, Comprehensive Assessment of Spoken Language (CASL-2) values, or Functional Communication Profile (FCP-R) values.
77. The method of clause 67, wherein the amelioration in the expressiveness of speech in the user with multiple sclerosis occurs, when the second metric is decreased from the first metric by the first predetermined margin or when the second metric is changed from the first metric by the second predetermined margin, and wherein the first metric and the second metric are at least one of WAB values, BDAE values, CETI values, ABA-2 values, DDK rate values, PCC-R values, FDA-2 values, or DIP values.
78. The method of clause 67, wherein the amelioration in the expressiveness of speech in the user with affective disorder occurs, when the second metric is decreased from the first metric by the first predetermined margin or when the second metric is changed from the first metric by the second predetermined margin, and wherein the first metric and the second metric are at least one of Hamilton Rating Scale for Depression (HAM-D) values.
79. The method of clause 67, wherein the amelioration in the expressiveness of speech in the user with schizophrenia occurs, when the second metric is decreased from the first metric by the first predetermined margin or when the second metric is changed from the first metric by the second predetermined margin, and wherein the first metric and the second metric are at least one of Motivation and Pleasure Scale-Self Report (MAP-SR) values, Social Effort and Conscientiousness Scale (SEACS) Social Effort values, or SEACS Social Conscientiousness values.
80. The method of clause 67, wherein the first metric is determined based on a corresponding speech classification of a plurality of speech classifications in a first session of the plurality of sessions, and wherein the second metric is determined based on the respective first respective speech classification in a second session of the plurality of sessions.

## Claims

1. A method of providing instructions for speech based on characteristics of verbal communications from users, comprising:
identifying, by one or more processors, a first audio sample of a first verbal communication from a user;
generating, by the one or more processors, a first plurality of speech characteristics for the first verbal communication using the first audio sample;
selecting, by the one or more processors, from a plurality of actions, an action to modify one or more of the first plurality of speech characteristics to define an utterance for the user; and
providing, by the one or more processors, an instruction presenting a message to prompt the user to perform the utterance defined by the action selected from the plurality of actions.

2. The method of claim 1, wherein determining the first speech classification further comprises determining the verbal communication is not able to be understood, and
wherein selecting the action further comprises selecting the action for the user to modify at least one of the first plurality of speech characteristics in the utterance.

3. The method of claim 1, wherein determining the first speech classification further comprises determining the verbal communication is able to be understood, and
wherein selecting the action further comprises selecting the action for the user to maintain one or more of the first plurality of speech characteristics in the utterance.

4. The method of claim 1, wherein the determining the first plurality of speech characteristics further comprises generating a score indicating a degree of severity of at least one of the first plurality of the speech characteristics, and
wherein providing the instruction further comprises providing the instruction including the message to identify the score for presentation to the user.

5. The method of claim 1, wherein the first plurality of speech characteristics further comprises a corresponding plurality of scores, each of the plurality of scores defined along a scale for a respective speech characteristic.

6. The method of claim 1, wherein the determining the first action further comprises determining the first speech classification based on at least one of: (i) an average of the plurality of scores, (ii) a weighted combination of the plurality of scores, (iii) a comparison with a dataset comprised of a second plurality of scores, (iv) a neural network model, or (v) a generative transformer model.

7. The method of claim 1, wherein the determining the first action further comprises applying a machine learning (ML) model to the first plurality of speech characteristics, wherein the ML model is established using a training dataset comprising a plurality of examples, each of the plurality of examples identifying (i) a respective second audio sample of a second verbal communication and (ii) a respective second classification from the plurality of speech classifications.

8. The method of claim 1, wherein providing the instruction further comprises providing the instruction including the message to identify at least one of (i) one or more of the first plurality of speech characteristics and (ii) the action to modify the utterance, and optionally
further comprising identifying, by the one or more processors, from a plurality of factors, a factor based on at least one of the first plurality of speech characteristics; and
wherein providing the instruction further comprises providing the message to identify the factor.

9. The method of claim 1, further comprising generating, by the one or more processors, for playback to the user, a second audio sample by modifying the first audio sample in accordance with the action.

10. The method of claim 1, wherein generating the second audio sample further comprises applying a speech synthesis model to the first audio sample and the action to generate the second audio sample.

11. The method of claim 1, further comprising:
determining, by the one or more processors, a second speech classification for a second verbal communication of the user based on a second plurality of speech characteristics, the second plurality of speech characteristics generated from a second audio sample identified at a time subsequent to provision of the instruction; and
determining, by the one or more processors, a progress metric based on a comparison between the first speech classification from prior to the instruction and the second speech classification subsequent to the provision of the instruction.

12. The method of claim 1, wherein the first plurality of speech characteristics further comprises at least one of: (i) respiration, (ii) phonation, (iii) articulation, (iv) resonance, (v) prosody, (vii) pitch, (viii) jitter, (ix) shimmer, (x) rhythm, (xi) pacing, or (xii) pausing.

13. The method of claim 1, further comprising:
identifying, by the one or more processors, a first video sample of a first non-verbal communication from the user, at least in partial concurrence with the first verbal communication;
determining, by the one or more processors, a first plurality of non-verbal characteristics of the first non-verbal communication using the first video sample, the first plurality of non-verbal characteristics including at least one of a gesture, a facial expression, an eye contact by the user; and
wherein determining the action further comprises determining the action based on the first plurality of non-verbal characteristics.

14. The method of claim 1, wherein the user is affected by at least one of a speech impairment or a language impairment, and is undergoing speech therapy at least partially concurrently with the provision of the instruction, and/or
wherein the user is affected by a disorder associated with a speech impairment and is on a medication for the disorder at least partially concurrently with the provision of the instruction.

15. A system for providing instructions for speech based on characteristics of verbal communications from users, comprising:
one or more processors coupled with memory, configured to perform the method of any one preceding claim.
